(19) Europäisches Patentamt
European Patent Office
Office européen des brevets

(11) Numéro de publication: **0 030 505 B1**

# (12) FASCICULE DE BREVET EUROPEEN

(45) Date de publication du fascicule du brevet: **24.07.85**

(51) Int. Cl.$^4$: **C 07 D 307/93,** C 07 D 405/04, C 07 B 57/00 // C07D207/16, C07C101/18

(21) Numéro de dépôt: **80401743.2**

(22) Date de dépôt: **05.12.80**

(54) **Nouvelles lactones substituées par un reste d'acide aminé, leur préparation et leur application au dédoublement de ces lactones et de ces acides aminés.**

(30) Priorité: **10.12.79 FR 7930201**

(43) Date de publication de la demande: **17.06.81 Bulletin 81/24**

(45) Mention de la délivrance du brevet: **24.07.85 Bulletin 85/30**

(84) Etats contractants désignés: **AT CH DE GB IT LI NL**

(56) Documents cités:
**EP-A-0 004 493**
**DE-A-2 510 714**
**DE-B-1 081 471**

(73) Titulaire: **ROUSSEL-UCLAF**
**35, boulevard des Invalides**
**F-75007 Paris (FR)**

(72) Inventeur: **Martel, Jacques**
**15, rue Douvillez**
**F-93140 Bondy (FR)**
Inventeur: **Tessier, Jean**
**30, rue Jean Moulin**
**F-94300 Vincennes (FR)**
Inventeur: **Teche, André**
**15, rue Godot de Mauroy**
**F-75009 Paris (FR)**

(74) Mandataire: **Tonnellier, Marie-José et al**
**ROUSSEL-UCLAF 102, route de Noisy**
**F-93230 Romainville (FR)**

**EP 0 030 505 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne de nouvelles lactones substituées par un reste d'acide aminé, leur préparation et leur application au dédoublement de ces lactones et de ces acides aminés.

L'invention a pour objet, sous toutes leurs formes stéréoisomères possibles ou sous forme de mélange de stéréoisomères, l'un quelconque des composés de formule générale (I)

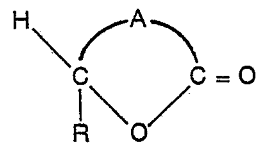

(I)

formule dans laquelle le symbole A représente une chaîne hydrocarbonée comportant de 1 à 10 chaînons, cette chaîne pouvant être linéaire, monocyclique ou bicyclique, ladite chaîne A comportant au moins un carbone asymétrique et dans laquelle les deux atomes ou radicaux différents qui substituent le ou les atomes de carbone asymétriques sont choisis indifféremment dans l'un ou l'autre des groupes suivants:
a) le groupe constitué par l'hydrogène, les atomes d'halogènes, le groupement nitro, les radicaux alcoyles comportant de 1 à 10 atomes de carbone, les radicaux cycloalcoyles comportant de 3 à 6 atomes de carbone, le radical phényle, les radicaux phényles substitués par au moins un des éléments du groupe constitué par les atomes d'halogènes, les radicaux alcoyles comportant de 1 à 6 atomes de carbone, le groupement carboxyle, le groupement nitrile, le groupement —CHO, le groupement:

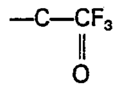

et les groupements S-alc et O-alc dans lesquels alc représente un groupement alcoyle comportant de 1 à 6 atomes de carbone,
b) le groupe constitué par les radicaux:

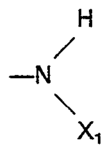

dans lesquels $X_1$ représente un atome d'hydrogène, un radical alcoyle comportant de 1 à 6 atomes de carbone.

le radical 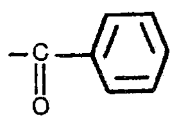 , le radical: 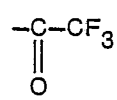 , le radical: 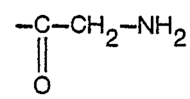 ,

le radical: 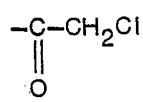 , ou un radical: 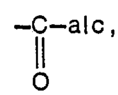,

dans lequel alc représente un radical alcoyle comportant de 1 à 6 atomes de carbone,
c) le groupe constitué par les radicaux:

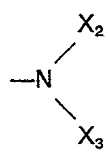

dans lequel $X_2$ et $X_3$, identiques ou différents, représentent un radical alcoyle comportant de 1 à 6 atomes de carbone ou bien dans lequel $X_2$ et $X_3$ représentent ensemble avec l'atomes d'azote auquel ils sont liés, un hétérocycle comportant 6 atomes ou bien $X_2$ représente un groupement carboxyle et $X_3$ représente un radical benzyle er R représente:
—soit le groupe ($III'_A$) de formule:

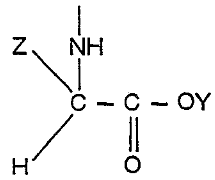

(III'$_A$)

formule dans laquelle Z représente le reste Z d'un acide aminé de formule (III$_1$)

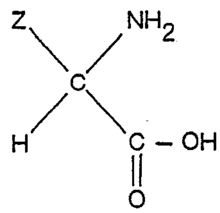

(III$_1$)

et Y représente un reste d'alcool primaire aliphatique comportant de 1 à 12 atomes de carbone, un reste d'alcool primaire cycloaliphatique comportant de 4 à 8 atomes de carbone ou un reste d'alcool benzylique,
—soit le groupe (III'$_B$) de formule:

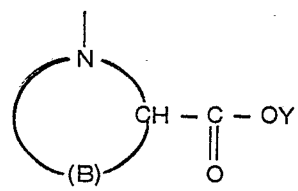

(III'$_B$)

formule dans laquelle B représente le reste B d'un acide aminé hétérocyclique comportant de 3 à 6 atomes de carbone de formule (III$_2$)

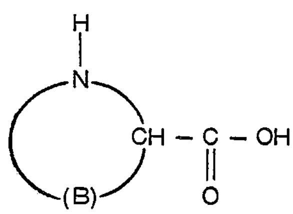

(III$_2$)

et Y conserve la signification précitée.
Ici et dans ce qui suit, les esters d'acides aminés de formule

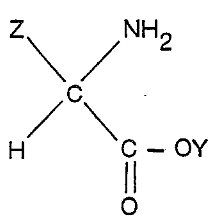

sont dénommés (III$_A$)
et les esters d'acides aminés cycliques de formule:

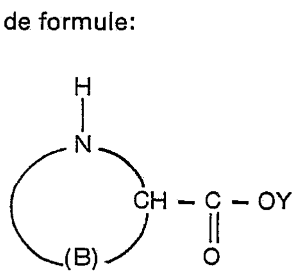

sont dénommés ($III_B$)

Les acides aminés (III)

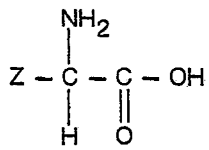 et 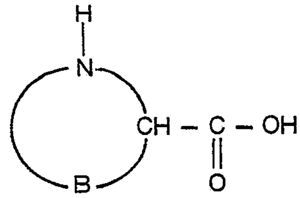

dont les restes figurent dans la formule (I) peuvent être des acides animés naturels, notamment ceux dont la liste figure dans les "Chemical Abstracts" Vol. 76 page 901.

Les acides aminés (III) peuvent également être des acides aminés synthétiques, notamment ceux qui figurent dans l'ouvrage "Amino acids, peptides and proteins volume 2 page 10 Specialist Periodic Reports. The Chemical Society Burlington House, London W1V 0BN, édition 1970 ou volume 1 page 13, édition 1969".

L'invention a particulièrement pour objet:

—les composés de formule (I) caractérisés en ce que la chaîne A est une chaîne hydrocarbonée aliphatique comportant 2 ou 3 atomes de carbone,

—les composés de formule (I) caractérisés en ce que la chaîne A est une chaîne hydrocarbonée aliphatique interrompue par un plusieurs hétéroatomes,

—les composés de formule (I) caractérisés en ce que la chaîne A est une chaîne hydrocarbonée aliphatique comportant une double liaison,

—les composés de formule (I) caractérisés en ce que la chaîne A est une chaîne hydrocarbonée monocyclique comportant de 3 à 6 atomes de carbone possédant éventuellement, une insaturation,

—les composés de formule (I) caractérisés en ce que la chaîne A est une chaîne hydrocarbonée bicyclique comportant de 5 à 10 atomes de carbone, possédant éventuellement une insaturation;

—les composés de formule (I) caractérisés en ce que la chaîne A a pour structure:

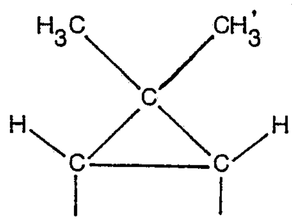

—les composés de formule (I) caractérisés en ce que la chaîne A a pour structure:

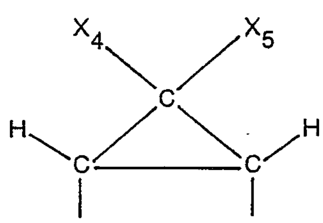

dans laquelle $X_4$ et $X_5$, identiques ou différents, représentent un atome d'hydrogène, de fluor, de chlore ou de brome ou un radical alcoyle comportant de 2 à 6 atomes de carbone ou bien $X_4$ et $X_5$ forment ensemble avec l'atome de carbone auquel ils sont liés, un homocycle carboné comportant de 3 à 7 atomes de carbone,

—les composés de formule (I) caractérisés en ce que la chaîne A a pour structure:

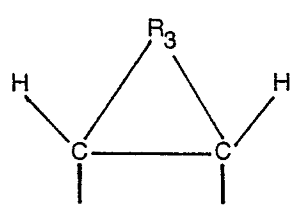

dans laquelle $R_3$ représente un atome d'oxygène, de soufre, un groupement —NH ou $NR_4$, $R_4$ étant un radical alcoyle comportant de 1 à 6 atomes de carbone.

L'invention a plus particulièrement pour object:

—les composés de formule générale (I) caractérisés en ce que R est choisi parmi les restes des esters d'acides aminés naturels dont les noms suivent: leucine, proline, phénylalanine, méthionine.

L'invention a plus précisément pour objet sous toutes leurs formes stéréoisomères possibles ou sous forme de mélange stéréoisomères, l'un quelconque des composés de formule générale (I) dont les noms suivent:

—(αR, 3R, 3aR,4S,7R,7aS) α-[(1-oxo 3a,4,7,7a-tétrahydro 1H, 3H-4,7-méthanoisobenzofuran-3-yl) amino]-isohexanoate de benzyle,

—(αS,3S,3aS,4R,7S,7aR) α[(1-oxo 3a, 4,7, 7a-tétrahydro 1H, 3H-4,7-méthanoisobenzofuran-3-yl)amino] isohexanoate de benzyle,

—(αR, 3S,3aS,4R,7S,7aR) α (1-oxo 3a,4,7, 7a-tétrahydro 1H, 3H-4,7-méthanoisobenzofuran 3-yl) D prolinate de benzyle,

—(αR,3R,3aR,4S,7R,7aS) α[1-oxo 3a, 4,7,7a-tétrahydro 1H, 3H-4,7-méthanoisobenzofuran 3-yl]L prolinate de benzyle,

—(αR,3R,3aR,4S,7R,7aS) α-[1-oxo 3a, 4,7, 7a-tétrahydro 1H, 3H-4,7 méthanoisobenzofuran-3-yl-amino]-phényl propanoate de méthyle,

—(αS,3S,3aS,4R,7S,7aR) α-[(1-oxo 3a,4,7, 7a-tétrahydro 1H, 3H-4,7-méthanoisobenzofuran-3-yl) amino]-phényl propanoate de méthyle,

—(3S,3aS,4R,7S,7aR) α (1-oxo 3a, 4,7, 7a-tétrahydro 1H, 3H-4,7-méthanoisobenzofuran-3-yl) L-méthioninate de méthyle,

—(3R,3aR,4S,7R,7aS) α (1-oxo 3a,4,7,7a-tétrahydro 1H,3H-4,7-méthanoisobenzofuran-3-yl)L-méthioninate de méthyle.

L'invention a également pour objet un procédé de préparation des composés de formule (I), caractérisé en ce que l'on fait réagir un composé de formule (II):

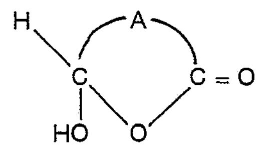

(II)

formule dans laquelle A conserve les significations précitées,

—soit avec un ester d'acide aminé de formule ($III_A$)

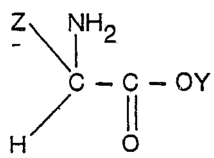

($III_A$)

—sout avec un ester d'acide aminé de formule ($III_B$)

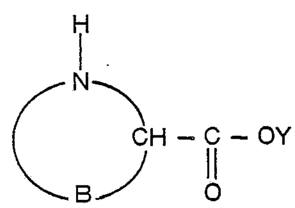

($III_B$)

formules dans lesquelles Y, Z et B conservent les significations précitées pour obtenir un composé de formule (I):

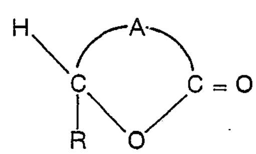

(I)

formule dans laquelle R et A conservent la signification précitée, produit de formule (I):

—ou bien dénommé ($I_A$) dans lequel les atomes chiraux possèdent une configuration bien définie, lorsque la copule lactonique et l'ester d'acide aminé possèdent un ou plusieurs atomes chiraux de configuration bien définie,

—ou bien dénommé ($I_B$) lorsqu'il s'agit d'un mélange de diastéréoisomères, la lactone étant un isomère optique bien défini et le ou les centres chiraux de l'ester d'acide aminé n'étant pas de configuration univoque,

—ou bien dénommé ($I_C$) lorsqu'il s'agit d'un mélange de diastéréoisomères, l'ester d'acide aminé étant un isomère optique bien défini et les atomes chiraux de la copule lactonique n'étant pas tous de configuration univoque, puis, le cas échéant, sépare par une méthode physique, les composés diastéréoisomères contenus ou bien dans les mélanges du type ($I_B$) ou bien dans les mélanges du type ($I_C$) dont les centres chiraux sont tous de configuration univoque.

L'invention a plus particulièrement pour objet un procédé de préparation des composés de formule (I) tel que défini précédemment, caractérisé en ce que l'eau formée lors de la condensation de l'ester d'acide aminé de formule ($III_A$) ou ($III_B$) et du composé lactonique (II) est éliminée par une méthode physique, notamment un procédé de préparation des composés (I) caractérisé en ce que l'eau formée est éliminée par décantation azéotropique au reflux d'un solvant choisi dans le groupe constitué par les solvants chlorés, les hydrocarbures aromatiques ou aliphatiques et les éthers et enfin un procédé de préparation des composés (I) tels que définis précédemment, caractérisé en ce que l'élimination de l'eau formée est obtenue en effectuant la réaction sous pression réduite.

Dans le procédé selon l'invention, la séparation des composés (I) diastéréoisomères est effectuée avantageusement par cristallisation ou chromatographie.

Comme composés lactoniques (II), on peut utiliser notamment le (1R,5S)6,6-diméthyl 4(R)-hydroxy 3-oxo bicyclo (3.1.0) hexan-2-one, la (1S,5R)6,6-diméthyl 4(S)-hydroxy 3-oxa bicyclo (3.1.0) hexan-2-one, la (3R,3aR,4S,7R,7aS)3-hydroxy tétrahydro 4,7-méthanoisobenzofuran-1-one, la (3S,3aS,4R, 7S,7aR)3-hydroxy tétrahydro 4,7-méthanoisobenzofuran-1-one.

Si le ou les atomes chiraux de la copule lactonique (II) sont chacun de configuration stérique déterminée (R) ou (S), lorsque le ou les atomes de carbone asymétriques de l'ester d'acide aminé ($III_A$) ou ($III_B$) sont aussi individuellement de configuration stérique déterminée (R) ou (S), on obtient directement avec rétention de configuration, les composés (I) dénommés ($I_A$).

Lorsque, dans le procédé selon l'invention, l'ester d'acide aminé ($III_A$) ou ($III_B$) possède un ou plusieurs carbones asymétriques non résolus, on obtient un mélange de composés (I) diastéréoisomères dénommés ($I_B$) que l'on peut alors séparer par un traitement physique notamment par chromatographie ou par cristallisation dans un solvant.

Ce dernier cas est particulièrement intéressant.

Après séparation des diastéréoisomères formant ($I_B$), par exemple, après séparation de ($I_A$), une simple hydrolyse, comme il est indiqué ci-après permet d'obtenir les esters d'acides aminés ($III_A$) ou ($III_B$) dédoublés au niveau du ou des atomes de carbone asymétriques racémiques qu'ils comportaient initialement. Lorsque l'ester d'acide aminé ($III_A$) ou ($III_B$) possède m centres chiraux non résolus, il se forme 2 m diastéréoisomères analogues à ($I_A$) qui peuvent éventuellement être séparés en individualités telles que ($I_A$).

On récupère, par ailleurs, le composé lactonique (II) utilisé dont le ou les atomes chiraux sont de configuration stérique déterminée (R) ou (S) telle qu'elle existait au départ.

Bien entendu, les considérations précédentes s'appliquent de la même façon aux esters d'acides aminés ($III_A$) et ($III_B$).

Le schéma 1 illustre le procédé énoncé précédemment.

Lorsque dans le procédé selon l'invention, la copule lactonique est de configuration racémique (RS) du fait d'une ou plusieurs centres chiraux non résolus, on obtient un mélange de composés (I) diastéréoisomères dénommés ($I_C$) que l'on peut alors séparer par un traitement physique, notamment par chromatographie ou par cristallisation dans un solvant.

Ce dernier cas est particulièrement intéressant. Après séparation des composés (I) diastéréoisomères formant ($I_C$), par exemple, après séparation de ($I_A$), une simple hydrolyse, comme il est indiqué ci-après permet d'obtenir le composé lactonique dédoublé au niveau du ou des atomes chiraux de configuration racémique (RS) qu'il comportait initialement. Lorsque la copule lactonique possède n centres chiraux non résolus, il se forme 2 n diastéréoisomères semblables à ($I_A$) qui peuvent éventuellement être séparés en individualités telles que ($I_A$).

On récupère, par ailleurs, l'ester d'acide aminé ($III_A$) ou ($III_B$) dont le ou les atomes de carbone asymétriques sont de configuration déterminée (R) ou (S) telles qu'elles existaient au départ dans l'acide aminé ($III_1$) ou ($III_2$).

Bien entendu, les considérations précédentes concernant les esters d'acides aminés ($III_A$) sont valables pour les esters d'acides aminés ($III_B$).

Le schéma 2 illustre le procédé précédent.

Dans tout ce qui précède, la présence d'un ou plusieurs centres chiraux dédoublés ou non dans les composés (II), ($III_1$) ou ($III_2$) implique les différentes possibilités suivantes:

—ou bien aucun des centres chiraux ne possède de configuration univoque soit (R) soit (S) et le composé (I) est alors un mélange de racémates groupant des énantiomères,

—ou bien une partie des centres chiraux possède une configuration univoque soit (R) soit (S) et le composé I est alors un mélange de diastéréoisomères,

—ou bien tous les centres chiraux ont une configuration univoque soit (R) soit (S) et le composé I est alors un isomère optique bien défini.

Il est à souligner que le seul atome de carbone chiral du composé lactonique indispensable au dédoublement des acides aminés (sous forme d'ester) ou au dédoublement de la lactone elle-même, est celui qui est situé en position α de l'oxygène endocyclique, le ou les autres atomes de carbone chiraux supplémentaires pouvant exister dans la chaîne latérale A, n'étant pas indispensables pour effectuer ces dédoublements.

L'invention a plus particulièrement pour objet un procédé de préparation des composés de formule (I) tel que défini précédemment, caractérisé en ce que le composé lactonique (II) est 1α(3R,3aR,4S,7R,7aS)-3-hydroxy tétrahydro 4,7-méthano isobenzofuran-1-one, un procédé de préparation des composés de formule (I) tel que défini précédemment, caractérisé en ce que le composé lactonique (II) est la (3S, 3aS, 4R, 7S, 7aR) 3-hydroxy tétrahydro 4,7-méthano isobenzofuran-1-one, ainsi qu'un procédé de préparation des composés de formule (I), caractérisé en ce que l'ester d'acide aminé est choisi dans le groupe constitué par le leucinate de benzyle, le prolinate de benzyle, le phénylalaninate de méthyle et le méthioninate de méthyle.

L'invention a également pour objet l'application des composés de formule générale (I) au dédoublement soit des composés de formule générale (II) soit des composés de formule générale (III), caractérisé en ce que l'on hydrolyse les composés diastéréoisomères isolés des mélanges du type $I_C$ ou $I_B$, par action d'un agent acide en milieu aqueux et,

—soit recueille le composé de formule générale (II) résultant dont le ou les carbones asymétriques sont de configuration univoque,

—soit soumet l'ester d'acide aminé de formule générale (III) résultant, à l'action d'un agent d'hydrolyse de la fonction ester puis recueille l'acide de formule générale (III) désiré, dont le ou les carbones asymétriques sont de configuration univoque.

En utilisant la méthode telle que décrite au schéma (3) dans lequel, au départ, l'ester d'acide aminé ($III_A$) ou ($III_B$) ne comportant qu'un seul centre chiral est un racémate formé de 2 antipodes (R) et (S), on peut, en lui opposant un isomère optique bien défini de la lactone (II), obtenir deux composés (I), du type ($I_A$), appelés respectivement ($I_D$) et ($I_E$) que l'on peut séparer par un traitement physique, notamment, par cristallisation ou chromatographie. Dans ces deux composés ($I_D$) et ($I_E$), le reste d'ester d'acide aminé présente une configuration antipodale. Par hydrolyse acide soit de ($I_D$), soit de ($I_E$), on récupère ainsi l'ester d'acide aminé ($III_A$) ou ($III_B$) dédoublé, respectivement, en ses antipodes (R) et (S), puis l'acide aminé ($III_1$) ou ($III_2$) lui- même, par action d'un réactif susceptible de libérer l'acide aminé de sa combinaison avec l'alcool. On peut notamment faire agir sur l'ester ($III_A$) ou ($III_B$), un agent alcalin en milieu aqueux. L'acide aminé ($III_1$) ou ($III_2$) est récupéré tel quel ou sous forme d'un de ses sels. On peut également effectuer une hydrogénolyse de l'ester ($III_A$) ou ($III_B$) à l'aide de l'hydrogène en présence d'un catalyseur. D'autres méthodes classiques sont également utilisables.

De manière analogue, par application de la méthode décrite au schéma (4), en utilisant au départ une lactone dont les centres chiraux présentent des configurations correspondant à un racémate et en lui opposant un isomère optique parfaitement défini d'un ester d'acide aminé ($III_A$) ou ($III_B$), on peut également obtenir deux composés (I), de type ($I_A$), appelés respectivement ($I_F$) et ($I_G$) que l'on peut séparer par une méthode physique, notamment par chromatographie ou par cristallisation.

Dans ces deux composés ($I_F$) et ($I_G$), le reste lactonique présente une configuration antipodale. Par hydrolyse acide soit de ($I^F$), soit de ($I^G$), on récupère la lactone (II) dédoublée en ses antipodes.

Dans le procédé de préparation des composés (I) selon l'invention ou dans l'application de ces composés, les esters d'acide aminé ($III_A$) ou ($III_B$) et les acides aminés ($III_1$) ou ($III_2$) sont commodément obtenus sous forme de sels, notamment sous forme de chlorhydrate.

En résumé, l'ensemble des réactions exposées ci-dessus permet, soit de dédoubler des acides aminés (après leur transformation en esters) à l'aide de lactones (II) de configuration bien définie, soit de dédoubler les lactones (II) à l'aide d'acides aminés (sous forme d'ester) de configuration bien définie.

Les acides aminés biologiquement utiles étant en général des composés optiquement actifs, la méthode de l'invention permet de dédoubler d'une façon avantageuse les acides aminés racémiques correspondants obtenus par synthèse.

Par ailleurs, le dédoublement des lactones (II), difficile à réaliser, trouve dans le procédé de l'invention une solution élégante.

Les exemples suivants illustrent l'invention sans la limiter.

## Exemple 1

(αR, 3R, 3aR, 4S, 7R, 7aS) α-[(1-oxo-3a,4,7,7a-tétrahydro 1H,3H-4,7-méthano isobenzofuran-3-yl)amino]isohexanoate de benzyle.

Pendant une heure et sous agitation, on chauffe au reflux en éliminant par azéotropie, l'eau formée, 1,66 g de (3R,3aR,4S,7R,7aS)3-hydroxy tétrahydro 4,7-méthano isobenzofuran-1-one $(\alpha)_D = +47°$ ($c$ =1% $CHCl_3$) 30 cm3 de benzène et 2,2 g de DL leucinate de benzyle.

On chasse le solvant sous pression réduite, cristallise le résidu dans environ 15 cm3 d'éther isopropylique, glace, essore à + 10°C et obtient 1,4 g de (αR,3R, 3aR, 4S, 7R, 7aS)α[(1-oxo 3a,4,7,7a-tétrahydro 1H, 3H-4,7-méthanoisobenzofuran 3-yl) amino] isohexanoate de benzyle. F = 88°C.

Spectre IR ($CHCl_3$)
NH: $3340^{cm^{-1}}$
C = O, γ lactone, ester: $1760^{cm^{-1}}$ à $1745^{cm^{-1}}$
les $CH_3$ géminés: $1390^{cm^{-1}}$—$1370^{cm^{-1}}$
phényle: $698^{cm^{-1}}$

Spectre de RMN ($CDCl_3$)
pic à 6,32 p p m attribué aux hydrogènes éthylèniques en 5 et 6 de la lactone,
pics à 1,32—1,45 p p m et 1,58—1,72 p p m attribués aux hydrogènes du $CH_2$ en 8 de la lactone,
pic à 4,68 p p m attribué à l'hydrogène en 3 de la lactone,
pic à 2,52 p p m attribué à l'hydrogène de l'amine,
pic à 0,85—0,93 p p m attribués aux hydrogènes des méthyles géminés,
pic à 5,2 p p m attribué aux hydrogènes en α du phényle,
pic à 7,42 p p m attribué aux hydrogènes aromatiques.
Dichroïsme circulaire (dioxane)
max à 225 nm $\Delta\varepsilon = +1,9$
max à 260 nm $\Delta\varepsilon = -0,03$

Le DL leucinate de benzyle utilisé au départ de l'exemple a été préparé de la façon suivante:

On agite pendant 10 minutes à 20°C, 3,93 g de tosylate de DL leucinate de benzyle, 25 cm3 d'eau démineralisée, 150 cm3 d'éther éthylique et 1,01 g de triéthylamine, extrait à l'éther, décante la phase organique, la séche, la concentre à sec sous pression réduite et obtient 2,2 g de produit attendu.

Exemple 2

(αS, 3S, 3aS, 4R, 7S, 7aR) α-[(1-oxo-3a, 4,7, 7a-tétrahydro 1H, 3H-4,7-méthano isobenzofuran-3-yl)-amino]-isohexanoate de benzyle.

En opérant comme à l'exemple 1, mais pendant 2 heures à reflux, avec 1,66 g de (3S, 3aS, 4R, 7S, 7aR) 3-hydroxytétrahydro 4,7-méthano isobenzofuran 1-one $[\alpha]_D$ −47° (*c* = 1% $CHCl_3$), 30cm3 de benzène et 2,2 g de DL leucinate de benzyle, on obtient 1,4 g de (αS, 3S, 3aS, 4R, 7S, 7aR) α-[(1-oxo-3a, 4,7 7a-tétrahydro 1H, 3H-4,7-méthano isobenzofuran-3-yl)-amino]isohexanoate de benzyle. F = 88°C.

Spectre IR ($CHCl_3$)
NH: $3340^{cm^{-1}}$
C = O, γ lactone, ester: $1760^{cm^{-1}}$ à $1745^{cm^{-1}}$
les $CH_3$ géminés: $1390^{cm^{-1}}$—$1370^{cm^{-1}}$
phényle: $698^{cm^{-1}}$

Spectre de RMN ($CDCl_3$)
pic à 6,18 p p m attribué aux hydrogènes éthylèniques en 5 et 6 de la lactone
pics à 1,32—1,45 p p m et 1,57—1,7 p p m attribués aux hydrogènes du $CH_2$ en 8 de la lactone,
pic à 4,6 p p m attribué à l'hydrogène en 3 de la lactone,
pic à 2,25 p p m attribué à l'hydrogène de l'amine,
pic à 0,83—0,95 p p m attribués aux hydrogènes des méthyles géminés,
pic à 5,1 p p m attribué aux hydrogènes en α du phényle,
pic à 7,35 p p m attribué aux hydrogènes aromatiques.
Dichroïsme circulaire (dioxane)
max 226 nm $\Delta\varepsilon = -1,86$
max vers 260 nm $\Delta\varepsilon \simeq +0,01$

Exemple 3

(αS, 3S, 3aS, 4R, 7S, 7aR) α (1-oxo 3a, 4,7, 7a-tétrahydro 1H,3H-4,7-méthano isobenzofuran-3-yl) D-prolinate de benzyle.

Stade A: Obtention du DL-prolinate de benzyle.

Pendant une heure à 20°C on agite 3,18 g de chlorhydrate de DL-prolinate de benzyle, 3 cm3 d'eau déminéralisée, 25 cm3 de benzène et 1,34 g de triéthylamine, décante, lave la phase organique à l'eau, la sèche, concentre à sec et obtient 2,6 g d'une huile incolore.

Stade B:

On mélange 2,2 g de (3S, 3aS, 4R, 7S, 7aR) 3-hydroxy tétrahydro 4,7-méthano isobenzofuran 1-one $[\alpha]_D = -47°$ (*c* = 1% $CHCl_3$), 25 cm3 de benzène anhydre et 2,6 g de DL prolinate de benzyle obtenu ci-dessus. Pendant environ 2 heures et demie et sous agitation, on porte le mélange au reflux en éliminant l'eau formée par entrainement azéotropique. On évapore le solvant sous pression réduite, recristallise dans l'éther isopropylique et obtient 1,5 g (αS, 3S, 3aS, 4R, 7S, 7aR) α (1-oxo 3a,4,7, 7a-tétrahydro 1H, 3H-4,7 méthano isobenzofuran-3-yl) D-prolinate de méthyle. F = 105°C.

Spectre IR ($CHCl_3$)
C = O $1760^{cm^{-1}}$ à $1740^{cm^{-1}}$
Aromatiques $1588^{cm^{-1}}$—$1495^{cm^{-1}}$
présence de C—O—C
phényle $696^{cm^{-1}}$

Dichroïsme circulaire (dioxane)
max~223 nm Δε = − 2,7
max 248 nm Δε = + 0,13
max 252 nm Δε = + 0,13

Spectre de RMN (deutérochloroforme)
pic à 6,07—6,4 p p m attribué aux hydrogènes éthyléniques en 5 — 6 de la lactone,
pics à 1,33—1,48 p p m et 1,58—1,73 p p m attribués aux hydrogènes du $CH_2$ en 8 de la lactone,
pic à 5,04 p p m attribué à l'hydrogène en 3 de la lactone,
pic à 3,75—3,87—3,98 p p m attribué à l'hydrogène en 2 de la proline
pic à 5,22 p p m attribué aux hydrogènes en α du phényle
pic à 7,37 p p m attribué aux hydrogènes aromatiques.

Exemple 4

(αR, 3R, 3aR, 4S, 7R, 7aS) α-(1-oxo-3a, 4,7, 7a-tétrahydro 1H, 3H-4,7-méthano isobenzofuran-3-yl) L-prolinate de benzyle.

On agite pendant une heure à 20°C, 2,4 g de chlorhydrate de DL prolinate de benzyle, 25 cm3 de benzène, 5 cm3 d'eau déminéralisée et 1,01 g de triéthylamine. On décante, lave la phase organique à l'eau, sèche, concentre à sec sous pression réduite et obtient 2,1 g de DL prolinate de benzyle. On ajoute 20 cm3 de benzène, 1,66 g de (3R, 3aR, 4S, 7R, 7aS) 3-hydroxy tétrahydro 4,7-méthano isobenzofuran-1-one $[\alpha]_D = + 47°$ ($c$ = 1% $CHCl_3$) et porte au reflux sous agitation pendant deux heures et demie, en éliminant par azéotropie l'eau formée. On concentre à sec sous pression réduite, essore, recristallise dans l'éther isopropylique et obtient 1,2 g d'(αR, 3R, 3aR, 4S, 7R, 7aS) α-(1-oxo 3a, 4,7, 7a-tétrahydro 1H, 3H-4,7-méthano isobenzofuran-3-yl) L-prolinate de benzyle,
F = 105°C.

Spectre IR ($CHCl_3$)
C = O: $1760^{cm^{-1}}$—$1740^{cm^{-1}}$
aromatiques: $1588^{cm^{-1}}$—$1495^{cm^{-1}}$
présence de C—O—C
phényle: $696^{cm^{-1}}$

Dichroïsme circulaire (dioxane)
max~220 nm Δε~ + 2,9
max 246 nm Δε − 0,19
infl 251 nm Δε + 0,18

Spectre de RMN (deutérochloroforme)
pics à 6—6,33 p p m attribués aux hydrogènes éthyléniques en 5—6 de la lactone
pics à 1,32—1,47 p p m et 1,58—1,73 p p m attribués aux hydrogènes du $CH_2$ en 8 de la lactone
pics à 4,95—5 p p m attribués à l'hydrogène en 3 de la lactone
pics à 3,72—3,83—3,95 p p m attribués à l'hydrogène en 2 de la proline
pics à 5,18 p p m attribué aux hydrogènes en α du phényle
pic à 7,4 p p m attribué aux hydrogènes aromatiques

Exemple 5

(αR, 3R, 3aR, 4S, 7R, 7aS) α-[(1-oxo-3a, 4,7, 7a-tétrahydro 1H, 3H-4,7-méthano isobenzofuran-3-yl)-amino]-phényl propanoate de méthyle.

Pendant 10 minutes à 20°C on agite 4,3 g de chlorhydrate du DL aminophénylpropanoate de méthyle, 43 cm3 d'eau déminéralisée, 86 cm3 d'éther éthylique et 3 cm3 de triéthylamine, décante la phase éthérée, extrait 2 fois à l'éther la phase aqueuse, réunit les phases organiques, sèche, concentre à sec sous pression réduite et obtient 3,5 g d'aminophénylpropanoate de méthyle. On ajoute 40 cm3 de benzène et 3,2 g de (3R, 3aR, 4S, 7R, 7aS) 3-hydroxy tétrahydro 4,7-méthano isobenzofuran 1-one $[\alpha]_D = + 47°$ ($c$ = 1% $CHCl_3$), porte au reflux en éliminant l'eau formée par entrainement azéotropique, pendant 16 heures sous agitation. On concentre à sec sous pression réduite, recristallise dans de l'isopropanol et obtient 2,2 g de (αR, 3R, 3aR, 4S, 7R, 7aR) α-[(1-oxo 3a, 4,7, 7a-tétrahydro 1H, 3H-4,7-méthano isobenzofuran-3-yl) amino] phényl propanoate de méthyle,
F = 120°C.

Exemple 6

(αS, 3S, 3aS, 4R, 7S, 7aR) α-[(1-oxo-3a, 4,7, 7a, tétrahydro 1H, 3H-4,7-méthano isobenzofuran-3-yl)amino]-phényl propanoate de méthyle.

On agite à 20°C pendant 10 minutes 4,3 g de chlorhydrate d'amino phényle propanoate de méthyle, 43 cm3 d'eau déminéralisée, 86 cm3 d'éther éthylique et 3 cm3 de triéthylamine. On extrait la phase aqueuse à l'éther, sèche la phase éthérée, concentre à sec sous pression réduite et obtient 8,3 g d'aminophényl propanoate de méthyle. On ajoute 3 g de (3S, 3aS, 4R, 7S, 7aR)3-hydroxy tétrahydro 4,7-méthano isobenzofuran-1-one $[\alpha]_D = -47°$ ($c = 1\%$ $CHCl_3$), 50 cm3 de benzène anhydre et porte au reflux sous agitation pendant une heure, en éliminant par azéotropie l'eau formée, puis concentre à sec sous pression réduite pour obtenir un résidu de 6,7 g. On recristallise dans l'isopropanol et obtient 2,1 g d'(αS, 3S, 3aS, 4R, 7S, 7aR) α[(1-oxo 3a, 4,7, 7a-tétrahydro 1H,3H 4,7-méthano isobenzofuran-3-yl)amino]phényl propanoate de méthyle.

F = 120°C.

Du filtrat on récupère 1,2 g de diastéréoisomère R après cristallisation dans l'éther isopropylique, F ≃ 77°C.

Spectre IR ($CHCl_3$)
C=O 1743 $^{cm-1}$
NH 3345 $^{cm-1}$

Spectre de RMN (deutéréochloroforme)
pics à 5,83—6,25 p p m attribués aux hydrogènes éthyléniques de la lactone,
pics à 1,27—1,42 p p m et 1,52—1,67 p p m attribués aux hydrogènes du $CH_2$ en 8 de la lactone,
pics à 2,33—3,33 p p m attribués aux hydrogènes en 3a et 7a de la lactone,
pics à 4,27—4,32 p p m attribués à l'hydrogène en 3 de la lactone,
pic à 3,75 p p m attribué à l'hydrogène en α de l'amine de la phènylalanine,
pics à 2,33—3,33 p p m attribués aux hydrogènes en α du phényle,
pic à 3,67 p p m attribué aux hydrogènes du $CH_3$,
pic à 7,35 p p m attribué aux hydrogènes aromatiques.

Exemple 7

Chlorhydrate de l'ester benzylique de D-leucine.

On agite pendant une heure à 20°C 2 g de (αR, 3R, 3aR, 4S, 7R, 7aS) α[(1-oxo-3a, 4,7, 7a-tétrahydro 1H,3H-4,7-méthano isobenzofuran-3-yl) amino] isohexanoate de benzyle obtenu à l'exemple 1, 15 cm3 d'eau déminéralisée et 1,5 cm3 d'acide chlorhydrique. On chauffe à environ 60°C pendant une heure puis laisse revenir à 20°C, ajoute 15 cm3 d'eau et extrait au chlorure de méthylène. La phase organique est lavée avec une solution de soude N puis à l'eau jusqu'à neutralité, séchée et concentrée à sec sous pression réduite. On ajoute 10 cm3 d'éther au résidu, fait barboter un léger courant d'acide chlorhydrique dans la solution. On isole par essorage le chlorhydrate de l'ester benzylique de D leucine formé, soit 800 mg.

F = 137°C.
$[\alpha]_D = +3°5$ (c = 1% HCl 0,1N)

Dichroïsme circulaire (dioxane)
max vers 217 nm $\Delta\varepsilon = -0,95$

Exemple 8

Chlorhydrate de l'ester benzylique de L-leucine.

On agite pendant une heure à 20°C 1 g de (αS, 3S, 3aS, 4R, 7S, 7aR) α-[(1-oxo-3a, 4,7, 7a tétrahydro 1H, 3H—4,7-méthano isobenzofuran-3-yl)amino] isohexanoate de benzyle obtenu à l'exemple 2, 8 cm3 d'eau déminéralisée et 0,8 cm3 d'acide chlorhydrique. On chauffe à 60°C pendant une heure, laisse refroidir à 20°C, dilue avec environ 15 cm3 d'eau et extrait au chlorure de méthylène. Les extraits organiques réunis sont lavés avec une solution de soude N puis à l'eau jusqu'à neutralité, sèchés et concentrés à sec sous pression réduite. On reprend le résidu par 10 cm3 d'éther et fait barboter un léger courant d'acide chlorhydrique dans la solution. On essore 350 mg de chlorhydrate de l'ester benzylique de L-leucine attendu.

F = 135°C.
$[\alpha]_D = -5,5° \pm 1$ (c = 1% HCl N/10)
Dichroïsme circulaire (dioxane): max à 219 nm $\Delta\varepsilon + 1,0$

Exemple 9

Chlorhydrate de D phénylalaninate de méthyle.

On agite à 20°C pendant 16 heures 2,2 g de (αR, 3R, 3aR, 4S, 7R, 7aR) α-[(1-oxo 3a,4,7, 7a-tétrahydro 1H,3H-4,7-méthano isobenzofuran-3-yl) amino]phényl propanoate de méthyle, obtenu à l'exemple 5, 20 cm3 d'eau déminéralisée et 1,7 cm3 d'acide chlorhydrique. On glace et essore l'insoluble. Le filtrat aqueux est saturé avec du carbonate de potassium. On récupère l'huile surnageante et extrait la solution aqueuse avec de l'éther. L'huile et les extraits éthérés réunis sont sèchés et concentrés à sec sous pression réduite.

On obtient 1,2 g d'une huile. On ajoute 20 cm3 de méthanol, refroidit à + 5, + 10°C et fait barboter un léger courant d'acide chlorhydrique. On concentre à sec sous pression réduite et obtient une huile épaisse que l'on cristallise dans l'éther. On obtient 750 mg de chlorhydrate de D phényl alaninate de méthyle, F = 160°C
$[\alpha]_D = \simeq 20° \pm 1°5$ (*c* = 1% Ethanol)

Spectre de RMN ($CDCl_3$)
pics à 3,35—3,43 p p m attribués aux hydrogènes en α du phényle,
pic à 4,42 p p m attribué à l'hydrogène en β du phényle,
pic à 3,7 p p m attribué aux hydrogènes du $CH_3$,
pic à 7,28 p p m attribué aux hydrogènes aromatiques.

## Exemple 10

L-phényl alaninate de méthyle.

On opère comme à l'exemple 9 avec 1,5 g de (αS, 3S, 3aS, 4R, 7S, 7aR) α[(1-oxo 3a, 4,7, 7a-tétrahydro 1H, 3H-4,7-méthano isobenzofuran-3-yl) amino] phenyl propanoate de méthyle obtenu à l'exemple 6, 5 cm3 d'eau et 1,1 cm3 d'acide chlorhydrique. On obtient 509 mg de L-phényle alaninate de méthyle sous forme d'huile.
$[\alpha]_D = + 28°5 \pm 1°$ (c = 1,5% éthanol)

Spectre de RMN ($CDCl_3$)
pics à 2,62—3,28 p p m attribués aux hydrogènes en α du phényle,
pics à 3,62—3,83 p p m attribués à l'hydrogène en β du phényle,
pic à 1,47 p p m attribué aux hydrogènes de l'amine,
pic à 3,67 p p m attribué aux hydrogènes du méthyle.

## Exemple 11

(3R, 3aR, 4S, 7R, 7aS) α(1-oxo 3a, 4,7, 7a-tétrahydro 1H,3H-4,7-méthano isobenzofuran-3-yl) L-méthioninate de méthyle.

Dans 10 cm3 de benzène, on introduit 0,5 cm3 de triéthylamine, 0,600 g de chlorhydrate d'ester méthylique de L-méthionine, agite une heure à 20°C, ajoute 0,500 g de (3R, 3aR, 4S, 7R, 7aS) 3-hydroxy tétrahydro-4,7-méthano isobenzofuran 1-one $[\alpha]_D = + 47°$ (*c* = 1% $CHCl_3$), porte le mélange réactionnel au reflux, l'y maintient pendant deux heures, en éliminant, par azéotropie, l'eau formée, refroidit, filtre, concentre le filtrat à sec par distillation sous pression réduite et obtient 1,13 g de (3R, 3aR, 4S, 7R, 7aS) α(1-oxo 3a, 4,7, 7a-tétrahydro 1H, 3H—4,7-méthano isobenzofuran-3-yl) L-méthioninate de méthyle.

Spectre IR (chloroforme)
absorption à 3348 $cm^{-1}$ attribuée au-NH;
absorption à 1743 $cm^{-1}$ attribuée aux carbonyles;
absorption à 1654 $cm^{-1}$ attribuée à —C = C et —C—O—C—.

Spectre de RMN (deutérochloroforme)
pic à 2,1 p p m attribué aux hydrogènes du méthyle de —S—$CH_3$
pic à 3,7 p p m attribué aux hydrogènes du méthyle du méthoxyle
pic à 4,7 p p m attribué à l'hydrogène en position 3 du cycle furanique
pic à 6,2 p p m attribué aux hydrogènes de la double liaison endocyclique

## Exemple 12

(3S, 3aS, 4R, 7S, 7aR) α (1-oxo 3a, 4,7, 7a tétrahydro 1H, 3H-méthano isobenzofuran-3-yl) L-méthioninate de méthyle.

Dans 10 cm3 de benzène on introduit 0,5 cm3 de triéthylamine et 0,600 g de chlorhydrate de l'ester méthylique de L-méthionine, agite pendant une heure à 20°C, ajoute 0,500 g de (3S, 3aS, 4R, 7S, 7aR) 3-hydroxy tétrahydro 4,7-méthano isobenzofuran-1-one $[\alpha]_D = - 47°$ (*c* = 1% $CHCl_3$), porte le mélange réactionnel au reflux, l'y maintient pendant deux heures, en éliminant, par décantation azéotropique, l'eau formée, refroidit, filtre, concentre le filtrat à sec par distillation sous pression réduite et obtient 1,18 g de (3S, 3aαS, 4R, 7S, 7aR) α(1-oxo 3a, 4,7, 7a tétrahydro 1H, 3H méthano isobenzofuran-3-yl) L-méthioninate de méthyle.

Spectre IR (chloroforme)
absorption à 3340 $cm^{-1}$ attribuée à —NH;
absorption à 1745 $cm^{-1}$ attribuée aux carbonyles;

Spectre de RMN (deutérochloroforme)
pic à 2,1 p p m attribué aux hydrogènes du méthyle de-S—$CH_3$,

pic à 3,7 p p m attribué aux hydrogènes du méthyle du méthoxyle,
pic à 4,6 p p m attribué à l'hydrogène en position 3 du cycle fumarique
pic à 6,23 p p m attribué aux hydrogènes de la double liaison endocyclique

Exemple 13

(3S, 3aS, 4R, 7S, 7aR) 3-hydroxy 3a, 4,7, 7a tétrahydro 1H, 3H-4,7-méthano isobenzofuran 1-one au départ de 3RS, 3aRS, 4RS, 7RS, 7aRS 3-hydroxy 3a, 4,7, 7a-tétrahydro 1H, 3H-4,7-méthano isobenzofuran-1-one.

Stade A: (αS, 3S, 3aS, 4R, 7S, 7aR) α-[(1-oxo 3a, 4,7, 7a-tétrahydro 1H,3H, 4,7, méthano isobenzofuran-3-yl)-amino]isohexanoate de benzyle.

Dans 40 cm3 de benzène anhydre on introduit 5,2 g de L-leucinate de benzyle, 4 g de (3RS, 3aRS, 4RS, 7RS, 7aRS) 3-hydroxy 3a, 4,7, 7a-tétrahydro 1H, 3H-4,7-méthano isobenzofuran-1-one, porte au reflux, maintient le reflux pendant une heure et trente minutes en éliminant l'eau formée, par décantation azéotropique, concentre à sec par distillation sous pression réduite, ajoute de l'éther isopropylique au résidu, isole par essorage le précipité formé et obtient 2,3 g de (αS, 3S, 3aS, 4R, 7S, 7aR) α[(1-oxo 3a,4,7, 7a-tétrahydro 1H, 3H-4,7-méthano isobenzofuran-3-yl)amino] isohexanoate de benzyle.
F = 95°C.

Stade B: (3S, 3aS, 4R, 7S, 7aR) 3-hydroxy 3a, 4,7, 7a-tétrahydro 1H, 3H-4,7 méthano isobenzofuran-1-one.

Dans 18 cm3 d'eau on introduit 1,8 cm3 de solution aqueuse concentrée d'acide chlorhydrique 22°Bé, ajoute 2,2 g de composé obtenu au stade A de l'exemple 13, (F = 95°C), porte le mélange réactionnel à 55°C, l'y maintient pendant 17 heures, refroidit à 20°C, extrait à l'acétate d'éthyle, lave la phase organique par une solution aqueuse 2N de soude, lave la phase aqueuse alcaline au chlorure de méthylène, à l'acétate d'éthyle, acidifie à pH 3,5 par addition d'une solution aqueous 2N d'acide chlorhydrique, extrait à l'acétate d'éthyle, lave la phase organique à l'eau la sèche, concentre à sec, ajoute de l'éther éthylique au résidu, isole par essorage le précipité formé, le sèche et obtient 0,450 g de (3S, 3aS, 4R, 7S, 7aR) 3-hydroxy 3a, 4,7, 7a tétrahydro 1H, 3H-4,7-méthano isobenzofuran 1-one.
F = 133°C.
$[\alpha]_D = -44°$ ($c$ = 1% benzène)

Exemple 14

D-Leucine

Dans un mélange de 20 cm3 d'acide acétique et 20 cm3 de méthanol, on introduit 0,5 g de chlorhydrate de l'ester benzylique de D-leucine (obtenu à l'exemple 7) et agite sous atmosphère d'hydrogène en présence de palladium jusqu'à fin d'absorption. On élimine le catalyseur par filtration, concentre le filtrat à sec sous pression réduite, chromatographie le résidu sur gel de silice et obtient la D-leucine.

Planches IV 1

SCHEMA 1

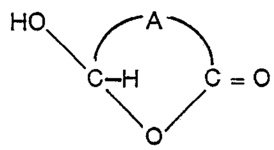

(II)

Isomère optique d'une lactone comportant un ou plusieurs centres chiraux de configuration univoque (R) ou (S)

Isomère optique d'un ester d'acide aminé RH $III_A$ ou $III_B$ comportant un ou plusieurs centres chiraux de configuration univoque (R) ou (S)

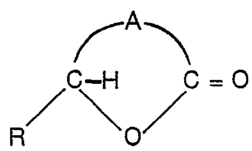

($I_A$)

avec rétention de configuration des restes de la lactone et de la molécule RH

Racémate d'un ester d'acide aminé RH ($III_A$ ou $III_B$) comportant un ou plusieurs centres chiraux non résolus

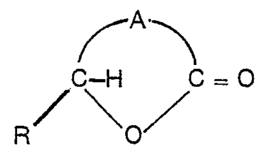

($I_B$)

Mélange de "m" diastéréoisomères dont ($I_A$) Le nombre m de diastéréoisomères peut être calculé comme suit:

RH

soit

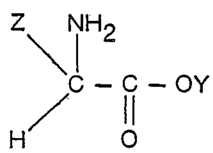

($III_A$)

soit

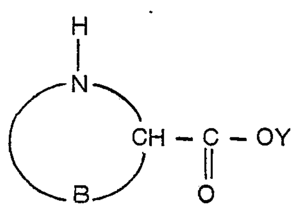

($III_B$)

$m = 2^X$ pour "X" centres chiraux non résolus dans l'acide aminé III

Traitement physique tel que chromatographie ou cristallisation

$I_A$ et m–1 autres diastéréoisomères

hydrolyse acide

esters d'acides aminés RH résolus ($III_A$ ou $III_B$)

hydrolyse acide de la fonction ester

acides aminés III résolus

Planches IV 2

SCHEMA 2

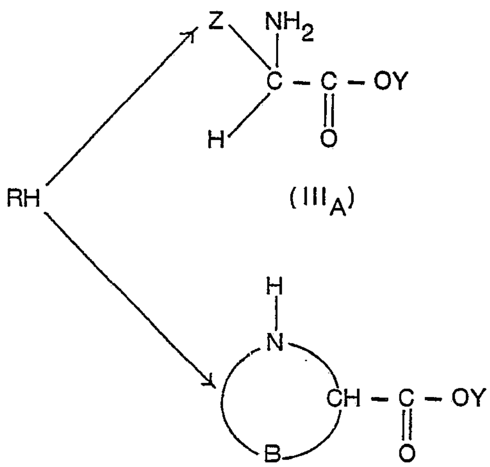

($III_A$)

($III_B$)

Isomère optique d'un ester d'acide aminé RH comportant un ou plusieurs centres chiraux de configuration univoque (R) ou (S)

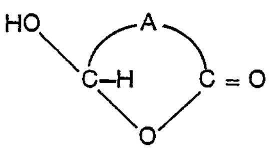

Isomère optique d'une lactone II comportant un ou plusieurs centres chiraux de configuration univoque (R) ou (S)

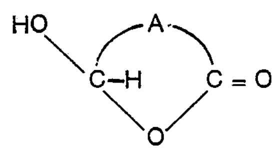

Racémate d'une lactone II comportant un ou plusieurs centres chiraux non résolus

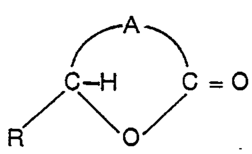

($I_A$)

avec rétention de configuration des restes de la lactone et de la molécule d'ester d'acide aminé RH

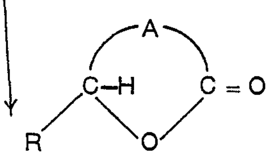

($I_C$)

Mélange de n diastéréoisomères dont $I_A$

Le nombre n de diastéréoisomères peut être calculé comme suit:

$n = 2^X$ pour "X" centres chiraux non résolus dans la lactone II

Traitement physique tel que chromatographie ou cristallisation

$I_A$ et n–1 autres diastéréoisomères

hydrolyse acide

lactones II résolues

SCHEMA 3

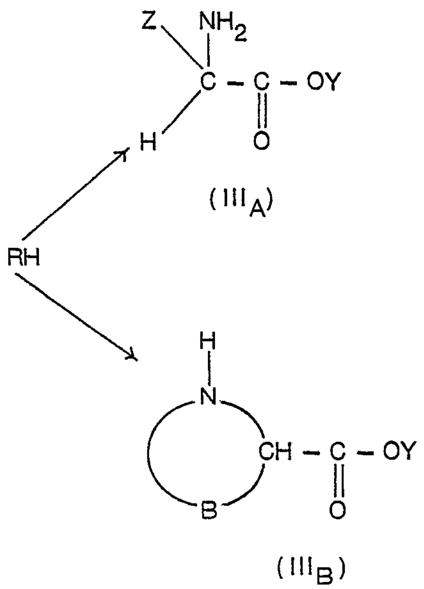

RH

($III_A$)

($III_B$)

RH ester d'acide aminé possédant un centre asymétrique non dédoublé

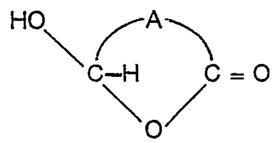

(II)

Isomère optique de lactone (II) possédant un ou plusieurs centres chiraux de configuration bien définie, soit (R), soit (S).

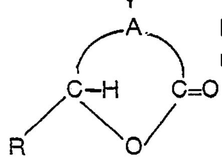

Mélange de deux diastéréoisomères ($I_D + I_E$) du type $I_A$

Séparation par traitement physique tel que chromatographie ou cristallisation.

$I_D$ (contenant un des deux isomères du reste d'ester d'acide aminé RH)

agent d'hydrolyse

isomère de l'ester d'acide aminé RH ou d'un de ses dérivés fonctionnels
(par exemple sous forme (S))

agent d'hydrolyse de la fonction ester

isomère de l'acide aminé (III) ou d'un de ses dérivés fonctionnels (par exemple sous forme (S))

$I_E$ (contenant l'autre isomère du reste d'ester d'acide aminé RH)

agent d'hydrolyse

l'autre isomère de l'ester d'acide aminé RH ou d'un de ses dérivés fonctionnels
(par exemple sous forme (R))

agent d'hydrolyse de la fonction ester

isomère de l'acide aminé (III) ou d'un de ses dérivés fonctionnels (par exemple sous forme (R))

Planches IV 4

SCHEMA 4

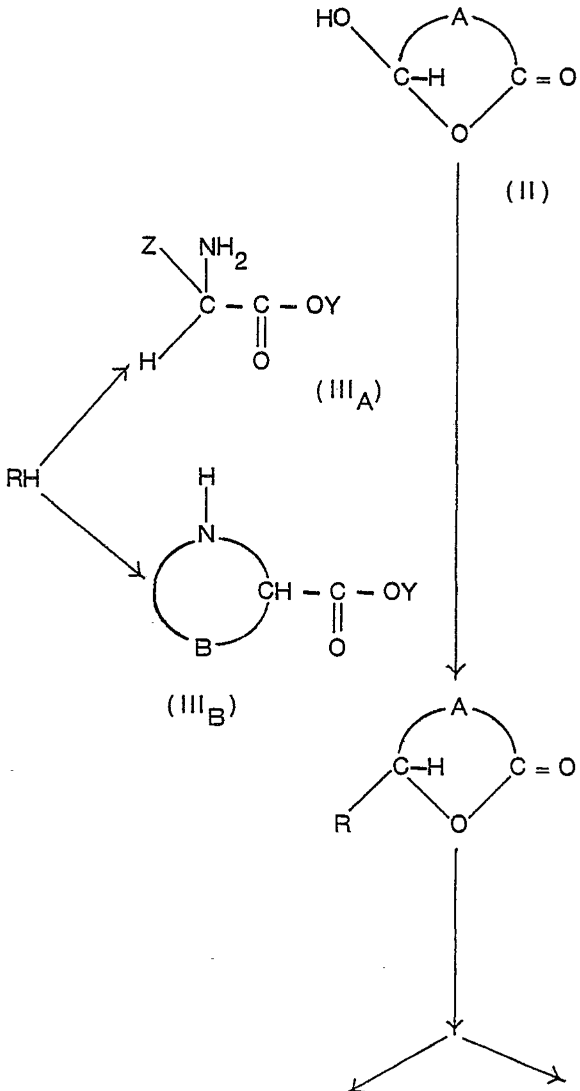

(II)

Lactone comprenant un centre chiral et qui compte tenu de la configuration de ce centre, se présente sous forme de racémique.

($III_A$)

Ester d'acide aminé RH Isomère optique d'une structure RH comportant un ou plusieurs centres chiraux de configurations univoques (R) ou (S).

($III_B$)

Mélange de deux diastéréoisomères ($I_F$ et $I_G$) du type $I_A$

Séparation par traitement physique (tel que chromatographie ou cristallisation)

$I_F$ contenant un des deux isomères du reste lactone

hydrolyse acide

isomère de la lactone

$I_G$ contenant l'autre isomère du reste lactone

hydrolyse acide

l'autre isomère de la lactone

**Revendications pour les Etats contractants: CH DE GB IT LI NL**

1. Sous toutes ses formes stéréoisomères possibles ou sous forme de mélange de stéréoisomères, l'un quelconque des composés de formule générale (I)

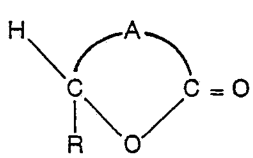

(I)

formule dans laquelle R représente
—soit le groupe ($III'_A$) de formule

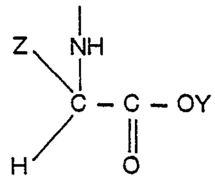

($III'_A$)

formule dans laquelle Z représente le reste Z d'un acide aminé de formule ($III_1$)

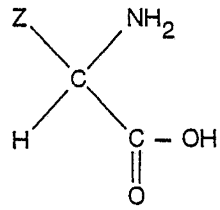

($III_1$)

et Y représente un reste d'alcool primaire aliphatique comportant de 1 à 12 atomes de carbone, un reste d'alcool primaire cycloaliphatique comportant de 4 à 8 atomes de carbone ou un reste d'alcool benzylique,
—soit le groupe ($III'_B$) de formule:

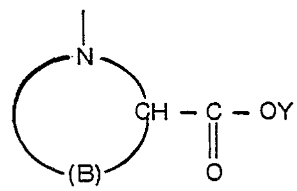

($III'_B$)

formule dans laquelle B représente le reste B d'un acide aminé hétérocyclique comportant de 3 à 6 atomes de carbone de formule ($III_2$)

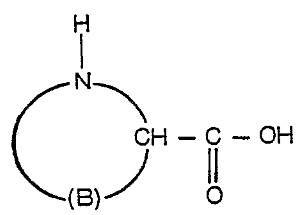

($III_2$)

et Y conserve la signification précitée, dans laquelle A représente une chaîne hydrocarbonée comportant de 1 à 10 chaînons, pouvant être linéaire, monocyclique ou bicyclique, ladite chaîne A comportant au moins un carbone asymétrique et dans laquelle les deux atomes ou radicaux différents qui substituent le ou les atomes de carbone asymétriques sont choisis indifféremment dans l'un ou l'autre des groupes suivants:
a) le groupe constitué par l'hydrogène, les atomes d'halogènes, le groupement nitro, les radicaux alcoyles comportant de 1 à 10 atomes de carbone, les radicaux cycloalcoyles comportant de 3 à 6 atomes de carbone, le radical phényle, les radicaux phényles substitués par un moins un des éléments du groupe constitué par les atomes d'halogènes, les radicaux alcoyles comportant de 1 à 6 atomes de carbone, le groupement carboxyle, le groupement nitrile, le groupement —CHO, le groupement:

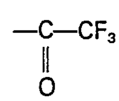

et les groupements S-alc et O-alc dans lesquels alc représente un groupement alcoyle comportant de 1 à 6 atomes de carbone,
b) le groupe constitué par les radicaux:

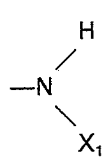

dans lesquels $X_1$ représente un atome d'hydrogène, un radical alcoyle comportant de 1 à 6 atomes de carbone.

le radical 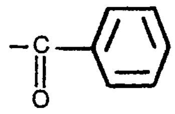 , le radical: 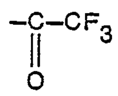 , le radical: 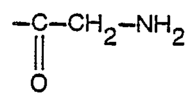 ,

le radical: 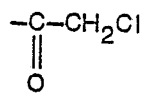 , ou un radical: 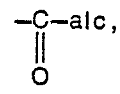

dans lequel alc représente un radical alcoyle comportant de 1 à 6 atomes de carbone,

c) le groupe constitué par les radicaux:

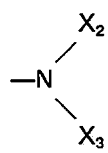

dans lequel $X_2$ et $X_3$, identiques ou différents, représentent un radical alcoyle comportant de 1 à 6 atomes de carbone ou bien dans lequel $X_2$ et $X_3$ représentent ensemble avec l'atome d'azote auquel ils sont liés, un hétérocycle comportant 6 atomes ou bien $X_2$ représente un groupement carboxyle et $X_3$ représente un radical benzyle.

2. Composés selon la revendication 1 caractérisés en ce que la chaîne A est une chaîne hydrocarbonée aliphatique comportant 2 ou 3 atomes de carbone.

3. Composés selon la revendication 1 caractérisés en ce que la chaîne A est une chaîne hydrocarbonée aliphatique interrompue par un ou plusieurs hétéroatomes.

4. Composés selon la revendication 1 caractérisés en ce que la chaîne A est une chaîne hydrocarbonée aliphatique comportant une double liaison.

5. Composés selon la revendication 1 caractérisés en ce que la chaîne A est une chaîne hydrocarbonée monocyclique comportant de 3 à 6 atomes de carbone, possédant éventuellement une insaturation.

6. Composés selon la revendication 1 caractérisés en ce que la chaîne A est une chaîne hydrocarbonée bicyclique comportant de 5 à 10 atomes de carbone possédant éventuellement une insaturation.

7. Composés selon la revendication 1, caractérisés en ce que la chaîne A a pour structure:

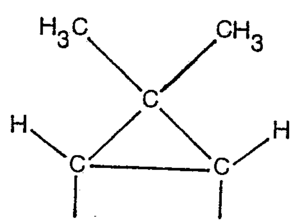

8. Composés selon la revendication 1, caractérisés en ce que la chaîne A a pour structure:

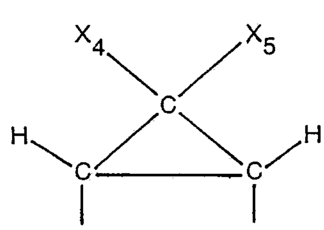

$X_4$ et $X_5$, identiques ou différents, représentant un atome d'hydrogène, de fluor, de chlore ou de brome ou un radical alcoyle comportant de 2 à 6 atomes de carbone ou bien $X_4$ et $X_5$ formant ensemble avec l'atome de carbone auquel ils sont liés, un homocycle carboné comportant de 3 à 7 atomes de carbone.

9. Composés selon la revendication 1, caractérisés en ce que la chaîne A a pour structure:

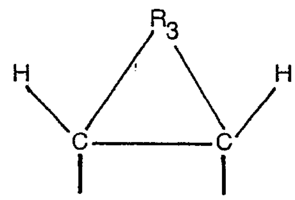

$R_3$ représentant un atome d'oxygène, de soufre, un groupement —NH ou —$NR_4$, $R_4$ étant un radical alcoyle comportant de 1 à 6 atomes de carbone.

10. Composés selon l'une quelconque des revendications 1 à 9 caractérisés en ce que R est choisi parmi les restes des esters d'acides aminés naturels dont les noms suivent: leucine, proline, phénylalanine, méthionine.

11. Les composés de formule I telle que définie à la revendication 1 dont les noms suivent:
—(αR, 3R, 3aR, 4S, 7R, 7aS) α-[(1-oxo 3a, 4,7, 7a-tétrahydro-1H, 3H-4,7-méthanoisobenzofuran-3-yl) amino] isohexanoate de benzyle,
—(αS, 3S, 3aS, 4R, 7S, 7aR) α-[(1-oxo 3a, 4,7, 7a-tétrahydro 1H, 3H-4,7-méthanoisobenzofuran-3-yl) amino] isohexanoate de benzyle,

12. Les composés de formule I telle que définie à la revendication 1 dont les noms suivent:
—(αR, 3S, 3aS, 4R, 7S, 7aSR) α [(1-oxo 3a,4,7, 7a-tétrahydro-1H, 3H-4,7-méthano isobenzofuran-3-yl) D prolinate de benzyle,
—(αR, 3R, 3aR, 4S, 7R, 7aS) α [1-oxo 3a, 4,7,7a-tétrahydro-1H, 3H-4,7-méthanoisobenzofuran 3-yl]L prolinate de benzyle,

13. Les composés de formule I telle que définie à la revendication 1 dont les noms suivent:
—(αR, 3R, 3aR, 4S, 7R, 7aR) α-[(1-oxo 3a, 4,7, 7a-tétrahydro-1H, 3H-4,7-méthanoisobenzofuran-3-yl) amino]phényl propanoate de méthyle,
—(αS, 3S, 3aS, 4R, 7S, 7aR) α-[(1-oxo 3a, 4,7 7a-tétrahydro 1H, 3H-4,7-méthanoisobenzofuran-3-yl) amino]phényl propanoate de méthyle,

14. Les composés de formule I telle que définie à la revendication 1 dont les noms suivent:
—(3S, 3aS, 4R, 7S, 7aR) α (1-oxo 3a, 4,7, 7a-tétrahydro-1H, 3H-4,7-méthanoisobenzofuran-3-yl)L-méthioninate de méthyle,
—(3R, 3aR, 4S, 7R, 7aS) α (1-oxo 3a, 4,7, 7a-tétrahydro-1H, 3H-4,7-méthanoisobenzofuran-3-yl)L-méthioninate de méthyle.

15. Procédé de préparation des composés de formule générale (I) tels que définis à la revendication 1, caractérisé en ce que l'on fait réagir un composé de formule (II):

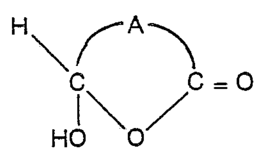

(II)

formule dans laquelle A conserve les significations de la revendication 1,
—soit avec un ester d'acide aminé de formule ($III_A$):

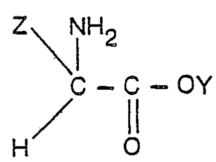

($III_A$)

—soit avec un ester d'acide aminé de formule ($III_B$):

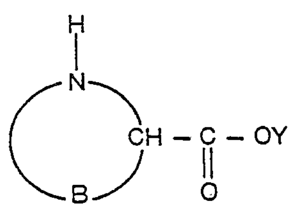

($III_B$)

formules dans lesquelles Y, Z et B conservent les significations de la revendication 1, pour obtenir un composé de formule (I):

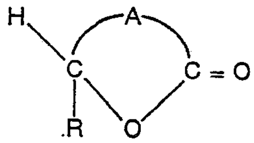

(I)

formule dans laquelle R et A conservent la signification précitée, produit de formule (I),
—ou bien dénommé($I_A$)dans lequel les atomes chiraux possédent une configuration bien définie, lorsque la copule lactonique et l'ester d'acide aminé possédent un ou plusieurs atomes chiraux de configuration bien définie,
—ou bien dénommé($I_B$)lorsqu'il s'agit d'un mélange de diastéréoisomères, la lactone étant un isomère optique bien défini et le ou les centres chiraux de l'ester d'acide aminé n'étant pas de configuration univoque,
—ou bien dénommé($I_C$)lorsqu'il s'agit d'un mélange de diastéréoisomères, l'ester d'acide aminé étant un isomère optique bien défini et les atomes chiraux de la copule lactonique n'étant pas tous de configuration univoque, puis, le cas échéant, sépare par une méthode physique, les composés diastéréoisomères contenus,
—ou bien dans les mélanges de type $I_B$,
—ou bien dans les mélanges du type $I_C$,
dont les centres chiraux sont tous de configuration univoque.

16. Procédé de préparation selon la revendication 15, caractérisé en ce que le composé lactonique (II) est choisi dans le groupe constitué par la (3R, 3aR, 4S, 7R, 7aS)3-hydroxy tétrahydro 4,7-méthanoisobenzofuran-1-one et la (3S, 3aS, 4R, 7s, 7aR)3-hydroxy tétrahydro 4,7-méthanoisobenzofuran-1-one,

17. Procédé selon la revendication 15 ou 16, caractérisé en ce que l'ester d'acide aminé est choisi dans le groupe constitué par le leucinate de benzyle, le prolinate de benzyle, le phényl alaninate de méthyle et le méthioninate de méthyle.

18. Procédé de préparation des composés de formule générale (I) selon la revendication 15, caractérisé en ce que l'eau formée lors de la condensation de l'ester d'acide aminé de formule ($III^A$) ou ($III^B$) et du composé lactonique (II) est éliminée par une méthode physique.

19. Procédé de préparation des composés de formule générale (I) selon la revendication 18, caractérisé en ce que l'eau formée est éliminée par décantation azéotropique au reflux d'un solvant choisi dans le groupe constitué par les solvants chlorés, les hydrocarbures aromatiques ou aliphatiques et les éthers et en ce que l'on opère, si désiré, sous pression réduite.

20. Procédé de préparation des composés de formule générale (I) selon l'une quelconque des revendications 15 à 19, caractérisé en ce que la séparation des composés I diastéréoisomères est effectuée par cristallisation ou chromatographie.

21. Application des composés de formule générale (I) tels que définis à la revendication 1 au dédoublement soit des composés de formule générale (II), soit des composés de formule générale (III), caractérisée en ce que l'on hydrolyse les composés diastéréoisomères isolés des mélanges du type ($I_C$) ou ($I_B$), par action d'un agent acide en milieu aqueux et,
—soit recueille le composé de formule générale (II) résultant dont le ou les carbones asymétriques sont de configuration univoque,
—soit soumet l'ester d'acide aminé de formule générale (III) résultant à l'action d'un agent d'hydrolyse de la fonction ester puis recueille l'acide de formule générale (III) désiré dont le ou les carbones asymétriques sont de configuration univoque.

**Revendications pour l'Etat contractant: AT**

1. Procédé de dédoublement des composés de formule (II):

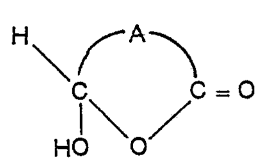

(II)

formule dans laquelle le symbole A représente une chaîne hydrocarbonée comportant de 1 à 10 chaînons, cette chaîne pouvant être linéaire, monocyclique ou bicyclique, ladite chaîne A comportant au moins un carbone asymétrique et dans laquelle les deux atomes ou radicaux différents qui substituent le ou les atomes de carbone asymétriques sont choisis indifféremment dans l'un ou l'autre des groupes suivants:
a) le groupe constitué par l'hydrogène, les atomes d'halogènes, le groupement nitro, les radicaux alcoyles comportant de 1 à 10 atomes de carbone, les radicaux cycloalcoyles comportant de 3 à 6 atomes de carbone, le radical phényle, les radicaux phényles substitués par un moins un des éléments du groupe

constitué par les atomes d'halogènes, les radicaux alcoyles comportant de 1 à 6 atomes de carbone, le groupement carboxyle, le groupement nitrile, le groupement —CHO, le groupement:

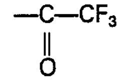

et les groupements S-alc et O-alc dans lesquels alc représente un groupement alcoyle comportant de 1 à 6 atomes de carbone,
b) le groupe constitué par les radicaux:

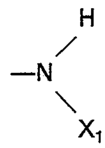

dans lesquels $X_1$ représente un atome d'hydrogène, un radical alcoyle comportant de 1 à 6 atomes de carbone.

le radical 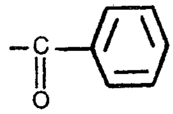 , le radical: 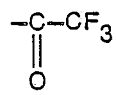 , le radical: 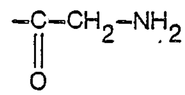 ,

le radical: 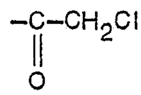 , ou un radical: 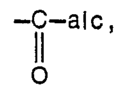,

dans lequel alc représente un radical alcoyle comportant de 1 à 6 atomes de carbone,
c) le groupe constitué par les radicaux:

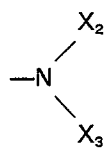

dans lequel $X_2$ et $X_3$, identiques ou différents, représentent un radical alcoyle comportant de 1 à 6 atomes de carbone ou bien dans lequel $X_2$ et $X_3$ représentent ensemble avec l'atome d'azote auquel ils sont liés, un hétérocycle comportant 6 atomes ou bien $X_2$ représente un groupement carboxyle et $X_3$ représente un radical benzyle, ainsi que des composés de formule (III):

R—H (III)

dans laquelle R représente:
—soit le groupe ($III'_A$) de formule:

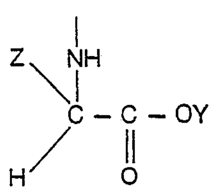

($III'_A$)

formule dans laquelle Z représente le reste Z d'un acide aminé de formule ($III_1$):

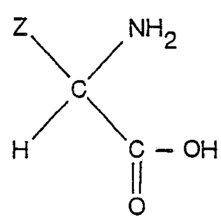

($III_1$)

—soit le groupe ($III'_B$) de formule:

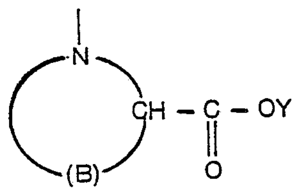

$(III'_B)$

formule dans laquelle B représente le reste B d'un acide aminé hétérocyclique comportant de 3 à 6 atomes de carbone de formule $(III_2)$:

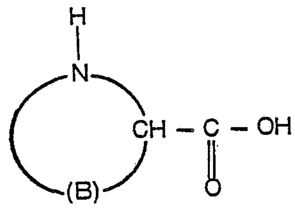

$(III_2)$

caractérisé en ce que l'on fait réagir un composé de formule (II):

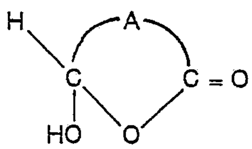

(II)

formule dans laquelle A conserve les significations précitées,

—soit avec un ester d'acide aminé de formule $(III_A)$:

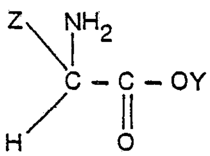

$(III_A)$

—soit avec un ester d'acide aminé de formule $(III_B)$:

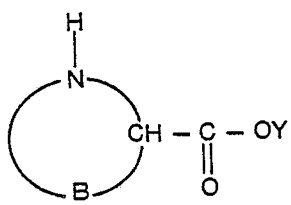

$(III_B)$

formules dans lesquelles Y représente un reste d'alcool primaire aliphatique comportant de 1 à 12 atomes de carbone, un reste d'alcool primaire cycloaliphatique comportant de 4 à 8 atomes de carbone ou un reste d'alcool benzylique et Z et B conservent les significations précitées, pour obtenir un composé de formule (I):

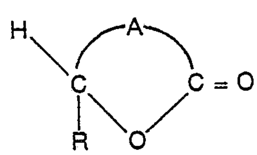

(I)

formule dans laquelle R et A conservent la signification précitée,

—ou bien dénommé$(I_B)$lorsqu'il s'agit d'un mélange de diastéréoisomères, la lactone étant un isomère optique bien défini et le ou les centres chiraux de l'ester d'acide aminé n'étant pas de configuration univoque,

—ou bien dénommé$(I^C)$lorsqu'il s'agit d'un mélange de diastéréoisomères, l'ester d'acide aminé étant un isomère optique bien défini et les atomes chiraux de la copule lactonique n'étant pas tous de configuration univoque, puis,

sépare par une méthode physique, les composés diastéréoisomères contenus,
—ou bien dans les mélanges de type $I_B$,
—ou bien dans les mélanges du type $I_C$,
dont les centres chiraux sont tous de configuration univoque, puis, hydrolyse les composés diastéréoisomères isolés des mélanges du type ($I_C$) ou ($I_B$), par action d'un agent acide en milieu aqueux et,
—soit recueille le composé de formule générale (II) résultant dont le ou les carbones asymétriques sont de configuration univoque,
—soit soumet l'ester d'acide aminé de formule générale (III) résultant à l'action d'un agent d'hydrolyse de la fonction ester puis recueille l'acide de formule générale (III) désiré dont le ou les carbones asymétriques sont de configuration univoque.

2. Procédé selon la revendication 1, caractérisé en ce que la chaîne A est choisie dans le groupe constitué par:
—une chaîne hydrocarbonée aliphatique comportant 2 ou 3 atomes de carbone,
—une chaîne hydrocarbonée aliphatique interrompue par un ou plusieurs hétéroatomes,
—une chaîne hydrocarbonée aliphatique comportant une double liaison,
—une chaîne hydrocarbonée monocyclique comportant de 3 à 6 atomes de carbone possédant éventuellement une insaturation,
—une chaîne hydrocarbonée bicyclique comportant de 5 à 10 atomes de carbone possédant éventuellement une insaturation,
—une chaîne de structure:

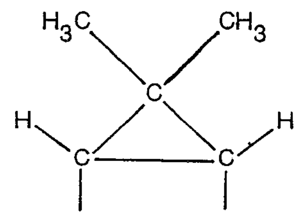

—une chaîne de structure:

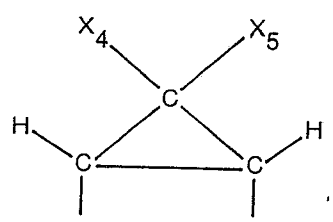

$X_4$ et $X_5$, identiques ou différents, représentant un atome d'hydrogène, de fluor, de chlore ou de brome ou un radical alcoyle comportant de 2 à 6 atomes de carbone ou bien $X_4$ et $X_5$ formant ensemble avec l'atome de carbone auquel ils sont liés, un homocycle carboné comportant de 3 à 7 atomes de carbone et une chaîne de structure:

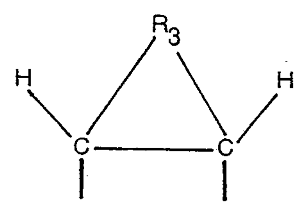

$R_3$ représentant un atome d'oxygène, de soufre, un groupement —NH ou —$NR_4$, $R_4$ étant un radical alcoyle comportant de 1 à 6 atomes de carbone.

3. Procédé selon la revendication 1 ou 2, caractérisé en ce que R est choisi parmi les restes des esters d'acides aminés naturels dont les noms suivent: leucine, proline, phénylalanine, méthionine.

4. Procédé selon la revendication 1, caractérisé en ce que le composé lactonique (II) est choisi dans le groupe constitué par la (3R, 3aR, 4S, 7R, 7aS)3-hydroxy tétrahydro 4,7-méthanoisobenzofuran-1-one et la (3S, 3aS, 4R, 7S, 7aR) 3-hydroxy tétrahydro 4,7-méthanoisobenzofuran-1-one.

5. Procédé selon la revendication 1 ou 4, caractérisé en ce que l'ester d'acide aminé est choisi dans le groupe constitué par le leucinate de benzyle, le prolinate de benzyle, le phényl alaninate de méthyle et le méthioninate de méthyle.

6. Procédé selon l'une quelconque des revendications 1 à 5, caractérisé en ce que l'eau formée lors de la condensation de l'ester d'acide aminé de formule ($III_A$) ou ($III_B$) et du composé lactonique (II), est éliminée par une méthode physique.

7. Procédé selon la revendication 6, caractérisé en ce que l'eau formée est éliminée par décantation azéotropique au reflux d'un solvant choisi dans le groupe constitué par les solvants chlorés, les hydrocarbures aromatiques ou aliphatiques et les éthers et, en ce que l'on opère, si désiré, sous pression réduite.

8. Procédé selon l'une quelconque des revendications 1 à 7, caractérisé en ce que la séparation des composés I diastéréoisomères est effectuée par cristallisation ou chromatographie.

**Claims for the Contracting States: CH DE GB IT LI NL**

1. In all their possible stereo-isomeric forms or in the form of a mixture of stereo-isomers, any one of the compounds with the general formula (I)

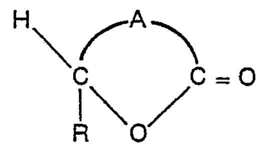

(I)

in which formula R represents
—either the group ($III'_A$) with the formula

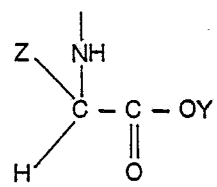

($III'_A$)

in which formula Z represents the residue Z of an amino acid with the formula ($III_1$),

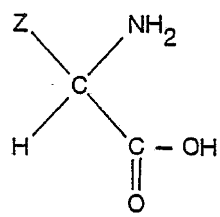

($III_1$)

and Y represents a residue of a primary aliphatic alcohol including from 1 to 12 carbon atoms, a residue of a primary cyclo-aliphatic alcohol including from 4 to 8 carbon atoms, or a residue of benzyl alcohol,
—or the group ($III'_B$), with the formula

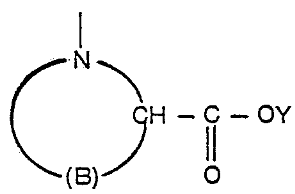

($III'_B$)

in which formula B represents the residue B of a heterocyclic amino acid including from 3 to 6 carbon atoms with the formula ($III_2$)

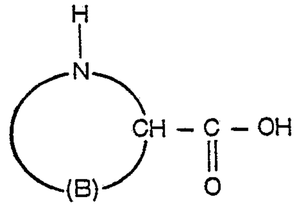

($III_2$)

and Y retains the previously stated significance, in which A represents a hydrocarbon chain including from 1 to 10 links which may be linear, monocyclic or bicyclic, the said chain A including at least one asymmetrical carbon and in which the two different atoms or radicals which substitute the asymmetrical atom or atoms of carbon are chosen indifferently from one or other of the following groups:
a) the group constituted by hydrogen, halogen atoms, the nitro group, alkyl radicals containing from 1 to 10 carbon atoms, the cycloalkyl radicals containing from 3 to 6 carbon atoms, the phenyl radical, phenyl radicals substituted by at least one of the elements of the group composed of the halogen atoms, the alkyl radicals including from 1 to 6 carbon atoms, the carboxyl group, the nitrile group, the —CHO group, the group:

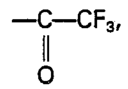

and the S-alk and O-alk groups in which alk represents an alkyl group containing from 1 to 6 carbon atoms,
b) the group constituted by the radicals:

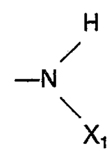

in which $X_1$ represents a hydrogen atom, an alkyl radical containing from 1 to 6 carbon atoms,

the radical 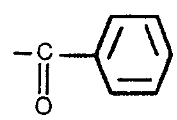 , the radical: 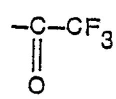 , the radical: 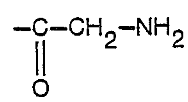 ,

the radical: 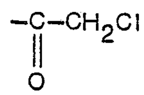 , or a radical: 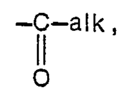 ,

in which alk represents an alkyl containing from 1 to 6 carbon atoms,
c) the group constituted by the radicals:

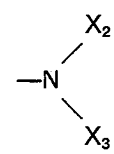

in which $X_2$ and $X_3$, being identical or different, each represent an alkyl radical containing from 1 to 6 carbon atoms or else in which $X_2$ and $X_3$ together represent, with the nitrogen atom to which they are attached, a heterocycle containing 6 atoms or else $X_2$ represents a carboxyl group and $X_3$ represents a benzyl radical.

2. Compounds according to Claim 1, characterized in that the chain A is an aliphatic hydrocarbon chain containing 2 or 3 carbon atoms.

3. Compounds according to Claim 1, characterized in that the chain A is an aliphatic hydrocarbon chain interrupted by one or more heteroatoms.

4. Compounds according to Claim 1, characterized in that the chain A is an aliphatic hydrocarbon chain containing a double bond.

5. Compounds according to Claim 1, characterized in that the chain A is a monocyclic hydrocarbon chain containing from 3 to 6 carbon atoms, possibly having an unsaturation.

6. Compounds according to Claim 1, characterized in that the chain A is a bicyclic hydrocarbon chain including from 5 to 10 carbon atoms, possibly having an unsaturation.

7. Compounds according to Claim 1, characterized in that the chain A has a structure:

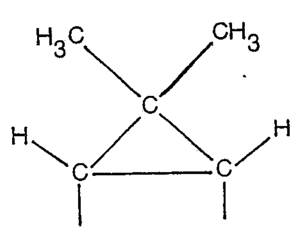

8. Compounds according to Claim 1, characterized in that the chain A has as structure:

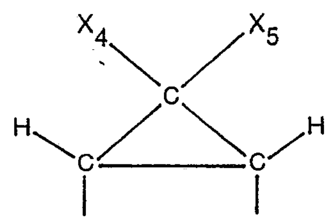

$X_4$ and $X_5$, being identical or different, each representing a hydrogen, fluorine, chlorine or bromine atom or an alkyl radical containing from 2 to 6 carbon atoms or else $X_4$ and $X_5$, together with the carbon atom to which they are bonded form a carbon homocycle containing from 3 to 7 carbon atoms.

9. Compounds according to Claim 1, characterized in that the chain A has as structure:

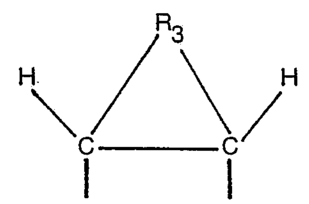

$R_3$ representing an oxygen or a sulphur atom, a —NH or —$NR_4$ group, $R_4$ being an alkyl radical containing from 1 to 6 carbon atoms.

10. Compounds according to any one of the Claims 1 to 9, characterized in that R is chosen from the residues of esters of natural amino acids, the names of which follow: leucine, proline, phenylalanine, methionine.

11. Compounds with the formula (I) as defined in Claim 1 of which the names follow:
Benzyl(αR,3R,3aR,4S,7R,7aS)α-[(1-oxo-3a,4,7,7a-tetrahydro-1H,3H-4,7-methanoisobenzofuran-3-yl)-amino]isohexanoate,
Benzyl(αS,3S,3aS,4R,7S,7aR)α-[(1-oxo-3a,4,7,7a-tetrahydro-1H,3H-4,7-methanoisobenzofuran-3-yl)-amino]isohexanoate.

12. Compounds with the formula (I) as defined in Claim 1 of which the names follow:
Benzyl(αR,3S,3aS,4R,7S,7aR)α-[1-oxo-3a,4,7,7a-tetrahydro-1H,3H-4,7-methanoisobenzofuran-3-yl]-D-prolinate.
Benzyl(αR,3R,3aR,4S,7R,7aS)α-[1-oxo-3a,4,7,7a-tetrahydro-1H,3H-4,7-methanoisobenzofuran-3-yl]-L-prolinate.

13. Compounds with the formula (I) as defined in Claim 1 of which the names follow:
Methyl(αR,3R,3aR,4S,7R,7aR)α-[(1-oxo-3a,4,7,7a-tetrahydro-1H,3H-4,7-methanoisobenzofuran-3-yl)-amino]phenylpropanoate.
Methyl(αS,3S,3aS,4R,7S,7aR)α-[(1-oxo-3a,4,7,7a-tetrahydro-1H,3H-4,7-methanoisobenzofuran-3-yl)-amino]phenylpropanoate.

14. Compounds with the formula (I) as defined, in Claim 1 of which the names follow:
Methyl(3S,3aS,4R,7S,7aR)α-(1-oxo-3a,4,7,7a-tetrahydro-1H,3H-4,7-methanoisobenzofuran-3-yl)L-methioninate.
Methyl(3R,3aR,4S,7R,7aS)α-(1-oxo-3a,4,7,7a-tetrahydro-1H,3H-4,7-methanoisobenzofuran-3-yl)L-methioninate.

15. Preparation process for the compounds with the general formula (I) as defined in Claim 1, characterized in that a compound with the formula (II):

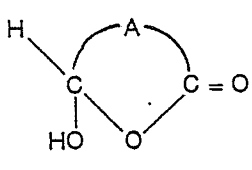
(II)

in which A retains the significances of Claim 1, is made to react
—either with an amino acid ester with the formula ($III_A$):

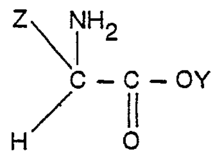

(III$_A$)

—or with an amino acid ester with the formula (III$_B$):

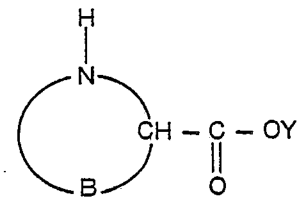

(III$_B$)

in which formulae Y, Z and B retain the significances of Claim 1, so as to obtain a compound with the formula (I):

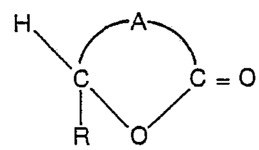

(I)

in which formula R and A retain the previously stated significance, which product with the formula (I)
—either is denoted by (I$_A$), in which the chiral atoms possess a well defined configuration, when the lactone copula and the ester of the amino acid possess one or more chiral atoms of well defined configuration
—or is denoted by (I$_B$) when it is a mixture of diastereo-isomers, the lactone being a well defined optical isomer and the chiral centre or centres of the amino acid ester not being of univocal configuration,
—or is denoted (I$_C$) when it is a mixture of diastereo-isomers, the amino acid ester being a well defined optical isomer and the chiral atoms of the lactone copula not all being of univocal configuration, then, if desired, the diastereo-isomeric compounds contained,
either in mixtures of type I$_B$,
or in mixtures of type I$_C$
in which the chiral centres are all of univocal configuration are separated by a physical method.

16. Preparation process according to Claim 15, characterized in that the lactone compound (II) is chosen from the group constituted by (3R,3aR,4S,7R,7aS)-3-hydroxytetrahydro-4,7-methanoisobenzofuran-1-one and (3S,3aS,4R,7S,7aR)-3-hydroxytetrahydro-4,7-methanoisobenzofuran-1-one.

17. Process according to Claim 15 or 16, characterized in that the amino acid ester is chosen from the group constituted by benzyl leucinate, benzyl prolinate, methylphenyl alaninate and methyl methioninate.

18. Preparation process for the compounds with the general formula (I) according to Claim 15, characterized in that the water formed during the condensation of the amino acid ester of formula (III$_A$) or (III$_B$) and of the lactone compound (II) is eliminated by a physical method.

19. Preparation process for the compounds with the general formula (I) according to Claim 18, characterized in that the water formed is eliminated by azeotropic decanting at the reflux of a solvent chosen from the group constituted by the chlorinated solvents, the aromatic or aliphatic hydrocarbides and the ethers and in that, if desired, the operation is carried out under reduced pressure.

20. Preparation process for the compounds with the general formula (I) according to any one of the Claims 15 to 19, characterized in that the separation of the diastereo-isomeric compounds (I) is effected by crystallization or chromatography.

21. Use of the compounds with the general formula (I) as defined in Claim 1 for the resolution either of the compounds with the general formula (II), or of the compounds with the general formula (III), characterized in that the diastereo-isomeric compounds isolated from the mixtures of the (I$_C$) or (I$_B$) type are hydrolyzed by the action of an acid agent in an aqueous medium and
—either the resulting compound with the general formula (II) is recovered, of which the asymmetrical carbons are of univocal configuration,
—or the amino acid ester with the general formula (III) resulting is submitted to the action of a hydrolyzing agent of the ester function, then the desired acid with the general formula (III) is recovered, of which the asymmetrical carbon or carbons are of univocal configuration.

**Claims for the Contracting State: AT**

1. Process for the resolution of compounds with the formula (II):

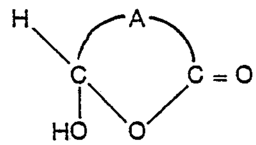 (II)

in which formula the symbol A represents a hydrocarbon chain containing from 1 to 10 links, this chain being able to be linear, monocyclic or bicyclic, the said chain A including at least one asymmetric carbon and in which the two different atoms or radicals which substitute the asymmetrical carbon atom or atoms are chosen indifferently from one or other of the following groups:

a) the group composed of hydrogen, the halogen atoms, the nitro group, the alkyl radicals containing from 1 to 10 carbon atoms, the cycloalkyl radicals containing from 3 to 6 carbon atoms, the phenyl radical, the phenyl radicals substituted by at least one of the elements or groups constituted by the halogen atoms, the alkyl radicals containing from 1 to 6 carbon atoms, the carboxyl group, the nitrile group, the —CHO group, the group:

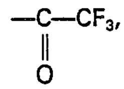

and S-alk and O-alk groups in which alk represents an alkyl group containing from 1 to 6 carbon atoms,

b) the group constituted by the radicals:

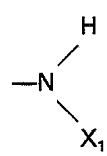

in which $X_1$ represents a hydrogen atom, an alkyl radical containing from 1 to 6 carbon atoms,

the radical 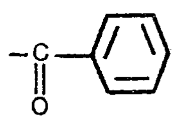, the radical: 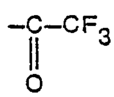, the radical: 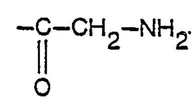,

the radical: 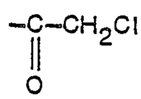, or a radical: 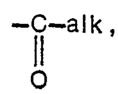,

in which alk represents an alkyl radical containing from 1 to 6 carbon atoms,

c) the group constituted by the radicals:

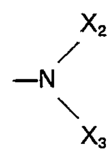

in which $X_2$ and $X_3$, identical or different, each represent an alkyl radical containing from 1 to 6 carbon atoms, or else in which $X_2$ and $X_3$ together with the nitrogen atom to which they are attached, represent a heterocycle containing 6 atoms or else $X_2$ represents a carboxyl group and $X_3$ represents a benzyl radical, as well as the compounds with the formula (III):

$$R—H \qquad (III)$$

in which R represents:

—either the groups ($III'_A$) with the formula:

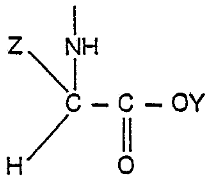

(III'$_A$)

in which Z represents the residue Z of an amino acid with the formula (III$_1$):

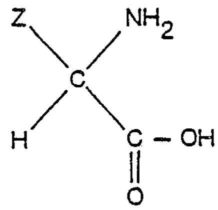

(III$_1$)

—or the group (III'$_B$) with the formula:

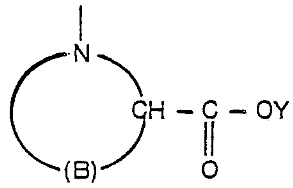

(III'$_B$)

in which formula B represents the residue B of a heterocyclic amino acid containing from 3 to 6 carbon atoms with the formula (III$_2$):

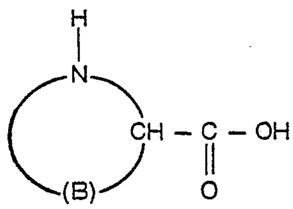

(III$_2$)

characterized in that a compound with the formula (II):

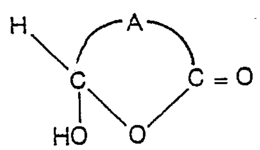

(II)

in which formula A retains the previously stated significance is made to react,
—either with an amino acid ester with the formula (III$_A$):

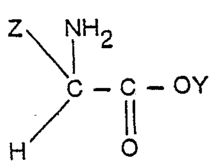

(III$_A$)

—or with an amino acid ester with the formula (III$_B$):

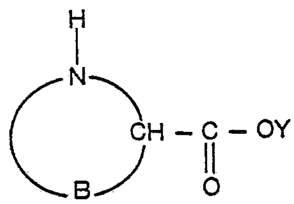

($III_B$)

in which formulae Y represents a residue of a primary aliphatic alcohol containing from 1 to 12 carbon atoms, a residue of a primary cycloaliphatic alcohol containing from 4 to 8 carbon atoms or the residue of benzyl alcohol and Z and B retain the previously stated significances, so as to obtain a compound with the formula (I):

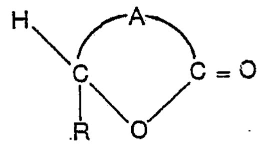

(I)

in which formula R and A retain the previously stated significance,
—or else denoted by ($I_B$) when it is concerned with a mixture of diastereo-isomers, the lactone being a well defined optical isomer and the chiral centre or centres of the amino acid ester not being of univocal configuration
—or else denoted by ($I_C$) when it is concerned with a mixture of diastereo-isomers, the amino acid ester being a well defined optical isomer and the chiral atoms of the lactone copula not all being of univocal configuration, then, the diastereo-isomeric compounds are separated by a physical method,
or else in mixtures of type ($I_B$)
or else in mixtures of type ($I_C$),
of which the chiral centres are all of univocal configuration, then the diastereo-isomeric compounds isolated from the mixtures of type ($I_C$) or ($I_B$), are hydrolysed by the action of an acid agent in an aqueous medium and,
—either the resulting compound with the general formula (II) is recovered, the asymmetric carbon or carbons of which are of univocal configuration,
—or the amino acid ester with the general formula (III) resulting is submitted to the action of a hydrolysing agent of the ester function, then the acid desired with the general formula (III) is recovered, of which the asymmetric carbon or carbons are of univocal configuration.

2. Process according to Claim 1, characterized in that the chain A is chosen from the group constituted by:
—an aliphatic hydrocarbon chain containing 2 or 3 carbon atoms,
—an aliphatic hydrocarbon chain interrupted by one or more hetero-atoms,
—an aliphatic hydrocarbon chain containing one double bond,
—a monocyclic hydrocarbon chain containing from 3 to 6 carbon atoms, possibly having one unsaturation,
—a bicyclic hydrocarbon chain containing from 5 to 10 carbon atoms, possibly having one unsaturation,
—a chain of structure:

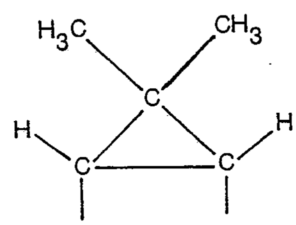

—a chain of structure:

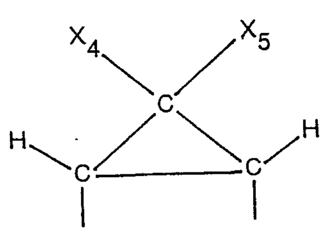

$X_4$ and $X_5$, being identical or different, representing each a hydrogen, fluorine, chlorine or bromine atom or an alkyl radical containing from 2 to 6 carbon atoms, or else $X_4$ and $X_5$, together with the carbon atom to which they are attached form a carbon homocycle containing from 3 to 7 carbon atoms, and a chain of structure:

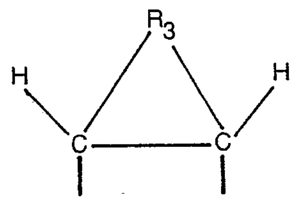

$R_3$ representing an oxygen or a sulphur atom, a group —NH or —$NR_4$, $R_4$ being an alkyl radical containing from 1 to 6 carbon atoms.

3. Process according to Claim 1 or 2, characterized in that R is chosen from the residues of the natural amino acid esters, the names of which follow: leucine, proline, phenylalanine, methionine.

4. Process according to Claim 1, characterized in that the lactone compound (II) is chosen from the group constituted by (3R,3aR,4S,7R,7aS)-3-hydroxytetrahydro-4,7-methano-isobenzofuran-1-one and (3S,3aS,4R,7S,7aR)-3-hydroxytetrahydro-4,7-methano-isobenzofuran-1-one.

5. Process according to Claim 1 or 4, characterized in that the amino acid ester is chosen from the group constituted by benzyl leucinate, benzyl prolinate, methylphenyl alaninate and methyl methioninate.

6. Process according to any one of the Claims 1 to 5, characterized in that the water formed during the condensation of the amino acid ester of formula ($III_A$) or ($III_B$) and of the lactone compound (II) is eliminated by a physical method.

7. Process according to Claim 6, characterized in that the water formed is eliminated by azeotropic decanting at the reflux of a solvent chosen from the group constituted by the chlorinated solvents, the aromatic or aliphatic hydrocarbides and the ethers and in that, if desired, the operation is carried out under reduced pressure.

8. Process according to any one of the Claims 1 to 7, characterized in that the separation of the diastereo-isomeric compounds (I) is done by crystallization or chromatography.

**Patentansprüche für die Vertragsstaaten: CH DE GB IT LI NL**

1. Eine beliebige Verbindung der allgemeinen Formel (I)

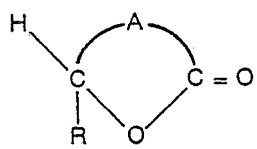

(I)

in allen ihren möglichen stereoisomeren Formen oder in der Form des Gemischs von Stereoisomeren, wobei in der Formel R bedeutet:

—entweder die Gruppe ($III'_A$) der Formel

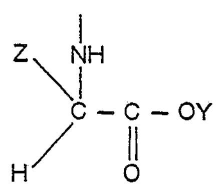

($III'_A$)

wobei in der Formel Z den Rest Z einer Aminosäure der Formel ($III_1$)

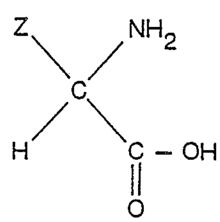

($III_1$)

bedeutet und Y einen primären aliphatischen Alkoholrest mit 1 bis 12 Kohlenstoffatomen, einen primären

cycloaliphatischen Alkoholrest mit 4 bis 8 Kohlenstoffatomen oder einen Benzylalkoholrest bedeutet, —oder die Gruppe ($III'_B$) der Formel

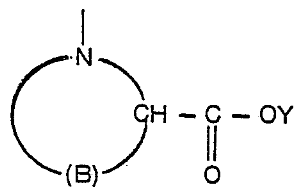

($III'_B$)

wobei in der Formel B den Rest B einer heterocyclischen Aminosäure mit 3 bis 6 Kohlenstoffatomen der Formel ($III_2$)

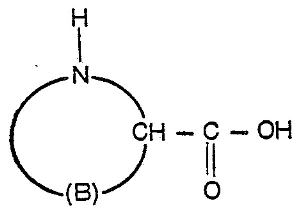

($III_2$)

bedeutet und Y die vorstehend angegebene Bedeutung beibehält, darstellt

worin A eine Kohlenwasserstoffkette mit 1 bis 10 Kettengliedern, die linear, monocyclisch oder bicyclisch sein kann, bedeutet, wobei die Kette A mindestens einen asymmetrischen Kohlenstoff enthält, und worin die beiden unterschiedlichen Atome oder Reste, die das oder die asymmetrischen Kohlenstoffatom(e) substituieren, unabhängig ausgewählt sind aus einer beliebigen der folgenden Gruppen:

a) der Gruppe, gebildet aus Wasserstoff, den Halogenatomen, der Nitrogruppe, den Alkylresten mit 1 bis 10 Kohlenstoffatomen, den Cycloalkylresten mit 3 bis 6 Kohlenstoffatomen, dem Phenylrest, den Phenylresten substituiert durch mindestens eines der Elemente aus der Gruppe der Halogenatome, der Alkylreste mit 1 bis 6 Kohlenstoffatomen, der Carboxylgruppe, der Nitrilgruppe, der Gruppe —CHO, der Gruppe

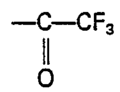

und den Gruppen S-Alk und O-Alk, worin Alk eine Alkylgruppe mit 1 bis 6 Kohlenstoffatomen darstellt,

b) der Gruppe, gebildet aus den Resten

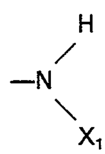

worin $X_1$ ein Wasserstoffatom, einen Alkylrest mit 1 bis 6 Kohlenstoffatomen bedeutet,

dem Rest 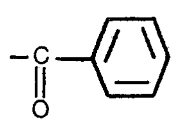 ,

dem Rest 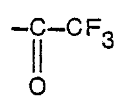 ,

dem Rest 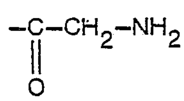 ,

dem Rest 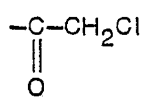 ,

oder einem Rest 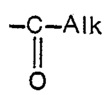 ,

worin Alk einen Alkylrest mit 1 bis 6 Kohlenstoffatomen bedeutet,
c) der Gruppe, gebildet durch die Reste

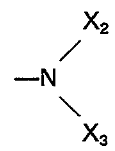

worin $X_2$ und $X_3$, die gleich oder verschieden sind, einen Alkylrest mit 1 bis 6 Kohlenstoffatomen bedeuten, oder auch worin $X_2$ und $X_3$ zusammen mit dem Stickstoffatom, an das sie gebunden sind, einen Heterozyklus mit 6 Atomen bilden, oder worin $X_2$ eine Carboxylgruppe bedeutet und $X_3$ einen Benzylrest bedeutet.

2. Verbindungen nach Anspruch 1, dadurch gekennzeichnet, daß die Kette A eine aliphatische Kohlenwasserstoffkette mit 2 oder 3 Kohlenstoffatomen ist.

3. Verbindungen nach Anspruch 1, dadurch gekennzeichnet, daß die Kette A eine aliphatische Kohlenwasserstoffkette ist, die durch ein oder mehrere Heteroatome unterbrochen ist.

4. Verbindungen nach Anspruch 1, dadurch gekennzeichnet, daß die Kette A eine aliphatische Kohlenwasserstoffkette ist, die eine Doppelbindung enthält.

5. Verbindungen nach Anspruch 1, dadurch gekennzeichnet, daß die Kette A eine monocyclische Kohlenwasserstoffkette mit 3 bis 6 Kohlenstoffatomen ist, die gegebenenfalls eine Unsättigung aufweist.

6. Verbindungen nach Anspruch 1, dadurch gekennzeichnet, daß die Kette A eine bicyclische Kohlenwasserstoffkette mit 5 bis 10 Kohlenstoffatomen ist, die gegebenenfalls eine Unsättigung aufweist.

7. Verbindungen nach Anspruch 1, dadurch gekennzeichnet, daß die Kette A die Struktur

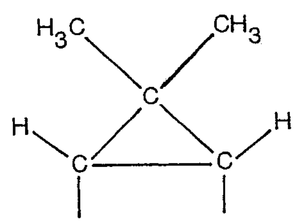

aufweist.

8. Verbindungen nach Anspruch 1, dadurch gekennzeichnet, daß die Kette A die Struktur

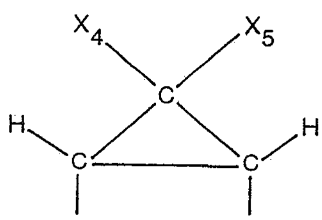

aufweist, wobei $X_4$ und $X_5$, die gleich oder verschieden sind, ein Wasserstoffatom, Fluor-, Chlor- oder Bromatom oder einen Alkylrest mit 2 bis 6 Kohlenstoffatomen bedeuten, oder $X_4$ und $X_5$ zusammen mit dem Atom, an das sie gebunden sind, einen Kohlenstoff-Homozyklus mit 3 bis 7 Kohlenstoffatomen bilden.

9. Verbindungen nach Anspruch 1, dadurch gekennzeichnet, daß die Kette A die Struktur

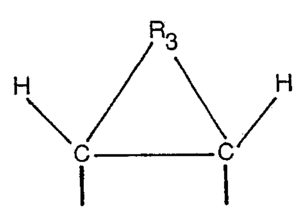

hat, worin $R_3$ ein Sauerstoff-, Schwefelatom, eine Gruppe —NH oder —$NR_4$ bedeutet, worin $R_4$ einen Alkylrest mit 1 bis 6 Kohlenstoffatomen darstellt.

10. Verbindungen nach einem der Ansprüche 1 bis 9, dadurch gekennzeichnet, daß der Rest R ausgewählt ist aus den Resten der Ester natürlicher Aminosäuren, deren Namen im folgenden aufgeführt sind: Leucin, Prolin, Phenylalanin, Methionin.

11. Die Verbindungen der Formel I, wie in Anspruch 1 definiert, deren Namen im folgenden aufgeführt sind:
—Benzyl-(αR,3R,3aR,4S,7R,7aS)-α-[(1-oxo-3a,4,7-7a-tetrahydro-1H, 3H-4,7-methanoisobenzofuran-3-yl)-amino]-isohexanoat,
—Benzyl-(αS,3S,3aS,4R,7S,7aR)-α-[(1-oxo-3a,4,7-7a-tetrahydro-1H, 3H-4,7-methanoisobenzofuran-3-yl)-amino]-isohexanoat.

12. Die Verbindungen der Formel I, wie in Anspruch 1 definiert, deren Namen im folgenden aufgeführt sind:
—Benzyl-(αR,3S,3aS,4R,7S,7aR)-α-[1-oxo-3a,4,7-7a-tetrahydro-1H, 3H-4,7-methanoisobenzofuran-3-yl]-D-prolinat,
—Benzyl-(αR,3R,3aR,4S,7R,7aS)-α-[1-oxo-3a,4,7-7a-tetrahydro-1H, 3H-4,7-methanoisobenzofuran-3-yl]-L-prolinat.

13. Die Verbindungen der Formel I, wie in Anspruch 1 definiert, deren Namen im folgenden aufgeführt sind:
—Methyl-(αR,3R,3aR,4S,7R,7aR)-α-[(1-oxo-3a,4,7-7a-tetrahydro-1H, 3H-4,7-methanoisobenzofuran-3-yl)-amino]-phenylpropanoat,
—Methyl-(αS,3S,3aS,4R,7S,7aR)-α-[(1-oxo-3a,4,7-7a-tetrahydro-1H, 3H-4,7-methanoisobenzofuran-3-yl)-amino]-phenylpropanoat.

14. Die Verbindungen der Formel I, wie in Anspruch 1 definiert, deren Namen im folgenden aufgeführt sind:
—Methyl-(3S,3aS,4R,7S,7aR)-α-(1-oxo-3a,4,7-7a-tetrahydro-1H, 3H-4,7-methanoisobenzofuran-3-yl)-L-methioninat,
—Methyl-(3R,3aR,4S,7R,7aS)-α-(1-oxo-3a,4,7-7a-tetrahydro-1H, 3H-4,7-methanoisobenzofuran-3-yl)-L-methioninat.

15. Verfahren zur Herstellung der Verbindungen der allgemeinen Formel (I), wie in Anspruch 1 definiert, dadurch gekennzeichnet, daß man eine Verbindung der Formel (II)

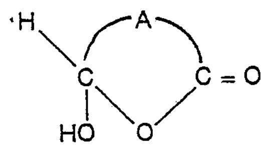

(II)

wobei in der Formel A die in Anspruch 1 angegebenen Bedeutungen beibehält, umsetzt mit
—entweder einem Aminosäureester der Formel ($III_A$)

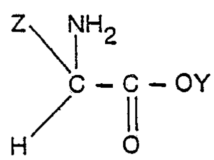

($III_A$)

—oder mit einem Aminosäureester der Formel ($III_B$)

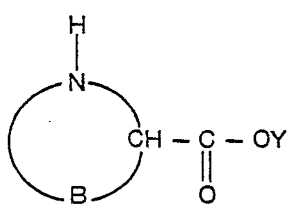

($III_B$)

wobei in den Formeln Y, Z und B die in Anspruch 1 angegebenen Bedeutungen beibehalten, unter Bildung einer Verbindung der Formel (I)

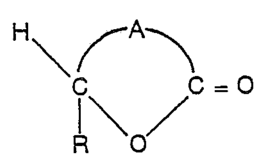

(I)

wobei in der Formel R und A die vorstehend angegebenen Bedeutungen beibehalten, das Produkt der Formel (I),
—oder auch bezeichnet als ($I_A$), worin die chiralen Atome eine definierte Konfiguration aufweisen, wenn die

Lactonbindung und der Aminosäureester ein oder mehrere chirale Atome mit definierter Konfiguration haben,

—oder auch bezeichnet als ($I_B$), wenn es sich um ein Gemisch der Diastereoisomeren handelt, wobei das Lacton ein definiertes optisches Isomeres ist und das chirale Zentrum oder die chiralen Zentren des Aminosäureesters keine eindeutige Struktur haben,

—oder als ($I_C$) bezeichnet, wenn es sich um ein Gemisch der Diastereoisomeren handelt, wobei der Aminosäureester ein definiertes optisches Isomeres ist und die chiralen Atome der Lactonbindung nicht alle eine eindeutige Konfiguration aufweisen,

worauf man gegebenenfalls nach einer physikalischen Methode in die enthaltenen diastereoisomeren Verbindungen

oder in die Gemische des Typs $I_B$,

oder in die Gemische des Typs $I_C$,

deren chiralen Zentren alle eine eindeutige Konfiguration aufweisen, trennt.

16. Herstellungsverfahren nach Anspruch 15, dadurch gekennzeichnet, daß die Lactonverbindung (II) ausgewählt wird aus der Gruppe von (3R,3aR,4S,7R,7aS)-3-Hydroxy-tetrahydro-4,7-methanoisobenzofuran-1-on und (3S,3aS,4R,7S,7aR)-3-Hydroxy-tetrahydro-4,7-methanoisobenzofuran-1-on.

17. Verfahren nach Anspruch 15 oder 16, dadurch gekennzeichnet, daß der Aminosäureester ausgewählt wird aus der Gruppe von Benzylleucinat, Benzylprolinat, Methylphenylalaninat und Methylmethioninat.

18. Verfahren zur Herstellung der Verbindungen der allgemeinen Formel (I) nach Anspruch 15, dadurch gekennzeichnet, daß das bei der Kondensation des Aminosäureesters der Formel ($III_A$) oder ($III_B$) und der Lactonverbindung (II) gebildete Wasser durch eine physikalische Methode entfernt wird.

19. Verfahren zur Herstellung der Verbindungen der allgemeinen Formel (I) nach Anspruch 18, dadurch gekennzeichnet, daß das Wasser durch azeotropes Dakantieren im Rückfluß, eines Lösungsmittels, ausgewählt aus der Gruppe von chlorierten Lösungsmitteln, aromatischen oder aliphatischen Kohlenwasserstoffen und den Ethern, entfernt wird, wobei man, falls gewünscht, unter verringertem Druck arbeitet.

20. Verfahren zur Herstellung der Verbindungen der allgemeinen Formel (I) gemäß einem der Ansprüche 15 bis 19, dadurch gekennzeichnet, daß die Trennung der diastereoisomeren Verbindungen I durch Kristallisation oder Chromatographie bewirkt wird.

21. Verwendung der Verbindungen der allgemeinen Formel (I), wie in Anspruch 1 definiert, zur Trennung von entweder Verbindungen der allgemeinen Formel (II) oder Verbindungen der allgemeinen Formel (III), dadurch gekennzeichnet, daß man die isolierten diastereoisomeren Verbindungen der Gemische des Typs ($I_C$) oder ($I_B$) hydrolysiert durch Einwirken eines sauren Mittels in wäßrigem Medium und

—entweder die resultierende Verbindung der allgemeinen Formel (II), deren asymmetrisches Kohlenstoffatom oder deren asymmetrische Kohlenstoffatome eindeutiger Konfiguration sind, sammelt,

—oder den resultierenden Aminosäureester der allgemeinen Formel (III) der Einwirkung eines Mittels zur Hydrolyse der Esterfunktion unterwirft, und dann die gewünschte Säure der allgemeinen Formel (III) sammelt, deren asymmetrischer Kohlenstoff oder deren asymmetrische Kohlenstoffe eine eindeutige Konfiguration haben.

**Patentansprüche für den Vertragsstaat: AT**

1. Verfahren zur Trennung der Verbindungen der Formel (II)

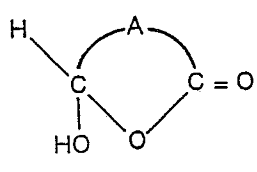

(II)

wobei in der Formel das Symbol A eine Kohlenwasserstoffkette mit 1 bis 10 Kettengliedern darstellt, wobei diese Kette linear, monocyclisch oder bicyclisch sein kann, wobei die Kette A mindestens einen asymmetrischen Kohlenstoff enthält, und worin die beiden unterschiedlichen Atome oder Reste, die das asymmetrische Kohlenstoffatom oder die asymmetrischen Kohlenstoffatome substituieren, unabhängig ausgewählt sind aus einer beliebigen der folgenden Gruppen:

a) der Gruppe, gebildet aus Wasserstoff, den Halogenatomen, der Nitrogruppe, den Alkylresten mit 1 bis 10 Kohlenstoffatomen, den Cycloalkylresten mit 3 bis 6 Kohlenstoffatomen, dem Phenylrest, den Phenylresten substituiert durch mindestens eines der Elemente der Gruppe der Halogenatome, der Alkylreste mit 1 bis 6 Kohlenstoffatomen, der Carboxylgruppe, der Nitrilgruppe, der Gruppe —CHO, der Gruppe

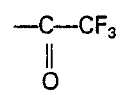

und den Gruppen S-Alk und O-Alk, worin Alk eine Alkylgruppe mit 1 bis 6 Kohlenstoffatomen darstellt, b) der Gruppe, gebildet durch die Reste

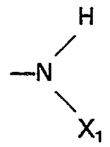

worin $X_1$ ein Wasserstoffatom, einen Alkylrest mit 1 bis 6 Kohlenstoffatomen darstellt,

dem Rest 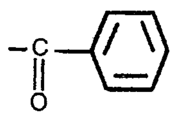 ,

dem Rest 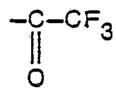 ,

dem Rest 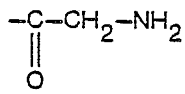 ,

dem Rest 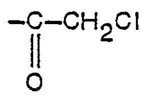 ,

oder einem Rest 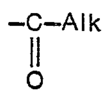 ,

worin Alk einen Alkylrest mit 1 bis 6 Kohlenstoffatomen bedeutet, c) der Gruppe, gebildet durch die Reste

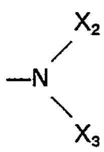

worin $X_2$ und $X_3$, die gleich oder verschieden sind, einen Alkylrest mit 1 bis 6 Kohlenstoffatomen darstellen, oder worin $X_2$ und $X_3$ zusammen mit dem Stickstoffatom, an das sie gebunden sind, einen Heterozyklus mit 6 Atomen bilden, oder $X_2$ eine Carboxylgruppe bedeutet und $X_3$ einen Benzylrest bedeutet, sowie der Verbindungen der Formel (III)

$$R—H \qquad (III)$$

worin R bedeutet:

—entweder die Gruppe ($III'_A$) der Formel

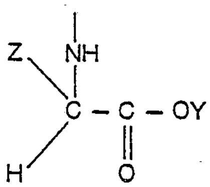

($III'_A$)

wobei in der Formel Z den Rest Z einer Aminosäure der Formel ($III_1$) bedeutet:

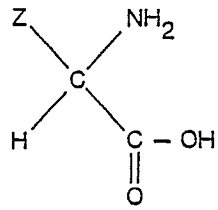

($III_1$)

—oder die Gruppe ($III'_B$) der Formel

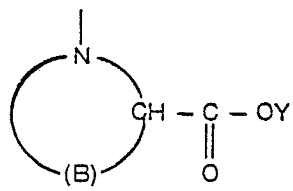

($III'_B$)

wobei in der Formel B den Rest B einer heterocyclischen Aminosäure mit 3 bis 6 Kohlenstoffatomen der Formel ($III_2$) bedeutet:

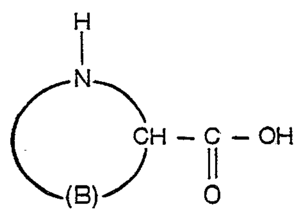

($III_2$)

dadurch gekennzeichnet, daß man eine Verbindung der Formel (II)

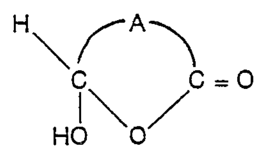

(II)

wobei in der Formel A die vorstehend genannten Bedeutungen beibehält, umsetzt mit
—entweder einem Aminosäureester der Formel ($III_A$)

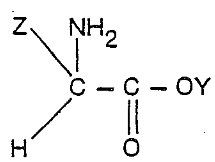

($III_A$)

—oder mit einem Aminosäureester der Formel ($III_B$)

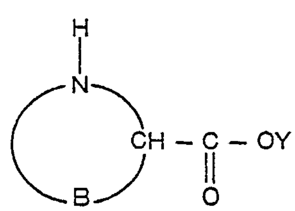

($III_B$)

wobei in den Formeln Y einen primären aliphatischen Alkoholrest mit 1 bis 12 Kohlenstoffatomen, einen primären cycloaliphatischen Alkoholrest mit 4 bis 8 Kohlenstoffatomen oder einen Benzylalkoholrest

bedeutet, und Z und B die vorstehenden Bedeutungen beibehalten, unter Erzielung einer Verbindung der Formel (I)

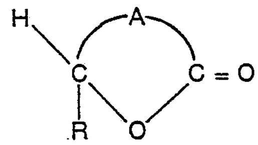

(I)

wobei in der Formel R und A die vorstehende Bedeutung beibehalten,

—oder auch bezeichnet als ($I_B$), wenn es sich um ein Gemisch der Diastereoisomeren handelt, wobei das Lacton ein definiertes optisches Isomeres ist und das chirale Zentrum oder die chiralen Zentren des Aminosäureesters keine eindeutige Konfiguration haben,

—oder auch bezeichnet als ($I_C$), wenn es sich um ein Gemisch der Diastereoisomeren handelt, wobei der Aminosäureester ein definiertes optisches Isomeres ist und die chiralen Atome der Lactonkombination keine eindeutige Konfiguration haben,

worauf man nach einer physikalischen Methode die enthaltenen Diastereoisomeren,

oder in die Gemische des Typs $I_B$,

oder in die Gemische des Typs $I_C$,

deren chiralen Zentren alle eine eindeutige Konfiguration haben, trennt, und anschließend die isolierten diastereoisomeren Verbindungen der Gemische des Typs ($I_C$) oder ($I_B$) hydrolysiert durch Einwirken eines sauren Mittels in wäßrigem Medium und

—die resultierenden Verbindungen der allgemeinen Formel (II), deren asymmetrischer Kohlenstoff oder deren asymmetrische Kohlenstoffe einheitliche Konfiguration haben, sammelt,

—oder den resultierenden Aminosäureester der allgemeinen Formel (III) der Einwirkung eines Mittels zur Hydrolyse der Esterfunktion unterwirft, und anschließend die gewünschte Säure der allgemeinen Formel (III) sammelt, deren asymmetrischer Kohlenstoff oder deren asymmetrische Kohlenstoffe eine eindeutige Konfiguration haben.

2. Verfahren nach Anspruch 1, dadurch gekennzeichnet, daß die Kette A ausgewählt ist aus der Gruppe, die gebildet wird durch:

—eine aliphatische Kohlenwasserstoffkette mit 2 oder 3 Kohlenstoffatomen,

—eine aliphatische Kohlenwasserstoffkette, unterbrochen durch ein oder mehrere Heteroatome,

—eine aliphatische Kohlenwasserstoffkette, die eine Doppelbindung enthält,

—eine monocyclische Kohlenwasserstoffkette mit 3 bis 6 Kohlenstoffatomen, die gegebenenfalls eine Unsättigung enthält,

—eine bicyclische Kohlenwasserstoffkette mit 5 bis 10 Kohlenstoffatomen, die gegebenenfalls eine Unsättigung enthält,

—eine Kette der Struktur

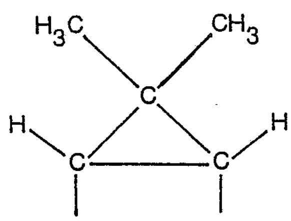

—eine Kette der Struktur

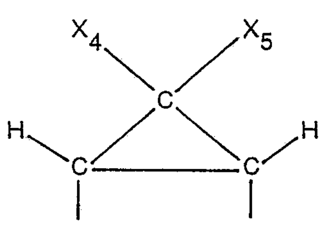

worin $X_4$ und $X_5$, die gleich oder verschieden sind, ein Wasserstoff-, Fluor-, Chlor- oder Bromatom oder einen Alkylrest mit 2 bis 6 Kohlenstoffatomen bedeuten, oder $X_4$ und $X_5$ zusammen mit dem Kohlenstoff-

atom, an das sie gebunden sind, einen Kohlenstoff-Homozyklus mit 3 bis 7 Kohlenstoffatomen bilden, und eine Kette der Struktur

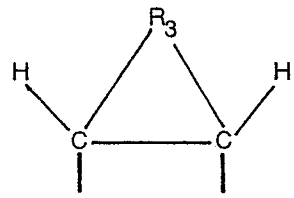

worin $R_3$ ein Sauerstoff-, Schwefelatom, eine Gruppe —NH oder —$NR_4$ bedeutet, worin $R_4$ einen Alkylrest mit 1 bis 6 Kohlenstoffatomen darstellt.

3. Verfahren nach Anspruch 1 oder 2, dadurch gekennzeichnet, daß R ausgewählt wird aus den Resten der Ester der natürlichen Aminosäuren, deren Namen im folgenden aufgeführt sind: Leucin, Prolin, Phenylalanin, Methionin.

4. Verfahren nach Anspruch 1, dadurch gekennzeichnet, daß die Lactonverbindung (II) ausgewählt wird aus der Gruppe, die gebildet wird durch (3R,3aR,4S,7R,7aS)-3-Hydroxy-tetrahydro-4,7-methanoisobenzofuran-1-on und (3S,3aS,4R,7S,7aR)-3-Hydroxy-tetrahydro-4,7-methanoisobenzofuran-1-on.

5. Verfahren nach Anspruch 1 oder 4, dadurch gekennzeichnet, daß der Aminosäureester ausgewählt wird aus der Gruppe von Benzylleucinat, Benzylprolinat, Methylphenylalaninat und Methylmethioninat.

6. Verfahren nach einem der Ansprüche 1 bis 5, dadurch gekennzeichnet, daß das während der Kondensation des Aminosäureesters der Formel ($III_A$) oder ($III_B$) und der Lactonverbindung (II) gebildete Wasser durch eine physikalische Methode entfernt wird.

7. Verfahren nach Anspruch 6, dadurch gekennzeichnet, daß das gebildete Wasser durch azeotropes Dekantieren unter Rückfluß von einem Lösungsmittel, ausgewählt aus der Gruppe, die gebildet wird durch chlorierte Lösungsmittel, aromatische oder aliphatische Kohlenwasserstoff und Ether, entfernt wird, und wobei man, falls gewünscht, unter verringertem Druck arbeitet.

8. Verfahren nach einem der Ansprüche 1 bis 7, dadurch gekennzeichnet, daß die Trennung der diastereoisomeren Verbindungen I durch Kristallisation oder Chromatographie erfolgt.